# EUROPEAN PATENT APPLICATION

(11) **EP 1 024 196 A1**
(43) Date of publication of application: **02.08.2000**
(21) Application number: 99400212.9
(22) Date of filing: 29.01.1999
(51) Int. Cl.: C12N 15/82, C12N 5/10, A01H 5/00

(54) **Nucleotide sequence having a tissue specific regulatory activity on the transcription of genes in plants**

(71) Applicant: KEYGENE N.V., 6700 AE Wageningen (NL)
(72) Inventor: Haring, Michel Albertus, 2024 RL Haarlem (NL); Cornelissen, Bernardus Johannes Clemens, 2361 EB Warmond (NL); Mes, Jurriaan Johannes, 6703 AV Wageningen (NL); Simons, Augustinus Franciscus Maria, 6715 JL Ede (NL)
(74) Representative: Desaix, Anne

(57) **Abstract**

The invention relates to a nucleotide sequence having a regulatory activity on the transcription of genes in plants and relates especially to a gene involved in the resistance mechanism of plants, to fungi, more particularly to soilborne fungi of the Fusarium species.

### Annex to the description

SEQUENCE LISTING

<110> KEYGENE N.V.

<120> NUCLEOTIDE SEQUENCE HAVING A TISSUE SPECIFIC REGULATORY
      ACTIVITY ON THE TRANSCRIPTION OF GENES IN PLANTS

<130> B4164_AD/CAL

<140>
<141>

<160> 5

<170> PatentIn Ver. 2.1

<210> 1
<211> 3823
<212> DNA
<213> Fusarium oxysporum

<400> 1
gaattcctgc aggtggaaat atacagccgt tgttagagaa acaacctttg gttatccaag 60

gtgctggagc aggaaatgaa ccttgcaaca ttttgactac tagtacatct caacattcat 120

cacaagacaa ttgactgaat cccgtgaaaa cttgctctcc gcctaatgaa tacagaacac 180

caatacttta gctttgtttt tatcattttc caaaaaaagt acatcaaaaa cagcatagcc 240

tcacgccatt ttgacttgta ctatacaacc tgacagcaca atcgcaaaag aaaagatgta 300

atcactagta catctcgaca tacaacatat ccaatgtaat ctcacaaatg gtatttcggg 360

aggataggat atacacaaat taaaattata tatatagaga gagagagtgt gtttttagag 420

ttgcaccgtt gtgctttgat aaaatacaga attttatgta taaaaaatta caacaggtta 480

tttctttata atttaacatc taataaagca tcttgtctca gttggtgaaa gtttatttcc 540

aataaaaata tgtcaattta gtggtatata tgtcagtcct caccgaagac caaactatat 600

ggtggtatgc atctattgat ttattgtaaa taaaaaatat atctgagttt gcttgataat 660

aaattattat gacgtgtttt aatttgcact atcaagaaga ctttattaca attttcaatc 720

attaagctcg aaaactctaa tcttatgtat aacaaaacaa ctacaaggga aaattgccat 780

agctctttgg tgtaatcaaa ttcccaactt gttgacgtgt tctcaagtat aaataaaaga 840

aaaattgtgt gtgagaagaa atgaaaataa aacattatga agacatagtg ttgttgttgg 900

gtgaaaagtg atacttttga ttttttttta aaaaggggga cagtgttttt ttattgtttt 960

ttcttctatg gctgtcccca tccctcacct acccactcaa aattattcta tttagataaa 1020

agactcttct tgttgtatga aaagtcatac tcatggaaaa gagtaggaaa aaggataatt 1080

tttgatatat ttattttatg tcctttaact ttaatattaa tgagtaaact tttgaatttg 1140

tataaagtta aataattaga tatatcaatc gtacgtaaca taaaattaga tgagggtata 1200

aaagccctac atgaggtgca tgtattgtta tatacattac gttataacac aaacagttga 1260

ggattgcaca acaacttgtg atcatcggat gcgagaagct cgtaaatggc cgaaataaat 1320

aacaacatac caagtataac ccgacaagtg ggatgtgtat atagacctta tcactatctt 1380

gtgggataga gggttgtttt cgatagaccc tcagctcaag taaacatagg gaaaaaatac 1440

cctagagaaa aggaaaatgg aacggatttc aattctttca cactgttact actatgaaaa 1500

taatggtcaa aatcgaactc aattgttaat tgcccaatat aggattgact actgattctt 1560

aaggaacgcg ttttcactac cttttgaaac cacaaaaaat ggacaaattc ctttccttag 1620

agacacatac ataaaacgag ctgaaatgtg ttttgtttct accagagttg ttcagattgc 1680

atcaatttca ctttcacatt cttgtttttt ttaattattt aaagttgcca gagctttgaa 1740

tctgatttaa attttgattt gttaaacatt gagtgttttt ttaattaggt ttcgtggacc 1800

tctaataata ggtcacttgt cagaaatgat ttacaaaact atattctttt tttgttaaaa 1860

aataatgata tgaatgaatt atttttaaaa tatatgtatt gtggataaat caaactttta 1920

tagaaatgac ataaataaat cattttaata aaattgtatc aaatgcttat tactattttc 1980

aaaaaatata tacactaagg gcccgtttgg atgggcttaa aaaaagcagc tttaaaaaaa 2040

gtactttcga aaggcgctga aacttatttt ttgaaataag cagttatgtg tttggaataa 2100

aagtgctgaa gttgctatgt caaacatgaa aagaggaaaa agggaaaaaa gagctanngt 2160

tagggttatn gtcgtaattt ggagattgta taaaaatatt aagggcaaaa aaaataaaaa 2220

tgtgtcaact taaaacagct tataagctaa aagttaaaag ctggggtaga ggtgtttttt 2280

ttttttttag cttataagtt gttttaagtt gaccacattt ttatttttct tgcccttaat 2340

atttttatac aatctcaaaa ttacgacata accctaacat ctttttctcc cattttttcc 2400

ttttcacgtt tgacatagca acttcagcac ttttatccaa acacataact gcttatttta 2460

aaaataagtt tcagcacttt caaaagtact tttttaaagc tgcttttatt aagcccatcc 2520

aaacgggccc taaaattgct aatgtttgct ctttctattc tcaaactccg taatatttaa 2580

gaaaatttgc taatgatagg tcacttttaa cactaaataa ttataaattg ggtagaaatt 2640

tatttatcat tttaagcttt tttaattttg agtcttctcc ctaattaaga cccttcccct 2700

cttgcttcaa ttatttaact gaatagtctt tgtcttattg ttgggtgaaa gtctgtcttc 2760

ttgttaggta ctaagtccta caataatatc aataatttgc tatggagaaa aaaatattat 2820

aggagaaaaa taattaattt taattcatga atatgtctta atatgcaact cattttgctt 2880

atatatatca aattaaactc tgttccttta actttttcct atgaagatac attttaattt 2940

atttgatgag gttagttttg aaatttatat tataataatg aaatgatata acttaaaaga 3000

agttgtttga tatcttatca gaatcatgca ggtactcata atataagaaa taattatgat 3060

gaaatttata tatgttttat gcagagattt attacgcatt gtttacttgg gttatgtatt 3120

acttatttca tcttttatca gaatgtaaaa ttatcattca ataagaaatc caattctgtt 3180

aaattcaaaa tacaaacaat aacattttca agaacgattt tttgcccaag aatatacagt 3240

aaacatattt atgatatggt aggtctcttt agtaattgac caacaaggat tgtggtggag 3300

tgggaaatac tctttaatac ttcaccaaga ggtctccaat ttgagcccct gaatacgaaa 3360

tcgtctttgt tagtatatac cctaacctaa tacaaaaatt agtatattag ccttcacagc 3420

taaaatcttt gtgacctgta agtcacgcga ggacaaattt accgtaacac caacttattc 3480

atgatataat tgtccctttt agcacggtaa taatgaggtg ggtagaaatt tattacttga 3540

gggccctttc tacacccacc cttattctct tgcttcaatt attgaattga agaagtaatg 3600

aaaaaacaga ctccattgga taaaggacag tttgcaaaca cagctgtaac aatttagagc 3660

actagcaaaa tagagagagt tttgagagaa atttttgttt gcaaattact cttaaccttc 3720

agcaggtaaa ataaagttct taactgagac tatttgaaga tatattttgt taaagaatca 3780

ttttgtgtgt ttccttgttt tgcttttgca gatttgagaa atg 3823

<210> 2
<211> 1334
<212> DNA
<213> Fusarium oxysporum

<400> 2
tactttttta aagctgcttt tattaagccc atccaaacgg gccctaaaat tgctaatgtt 60

tgctctttct attctcaaac tccgtaatat ttaagaaaat ttgctaatga taggtcactt 120

ttaacactaa ataattataa attgggtaga aatttattta tcattttaag cttttttaat 180

tttgagtctt ctccctaatt aagacccttc ccctcttgct tcaattattt aactgaatag 240

tctttgtctt attgttgggt gaaagtctgt cttcttgtta ggtactaagt cctacaataa 300

tatcaataat ttgctatgga gaaaaaaata ttataggaga aaaataatta attttaattc 360

atgaatatgt cttaatatgc aactcatttt gcttatatat atcaaattaa actctgttcc 420

tttaactttt tcctatgaag atacatttta atttatttga tgaggttagt tttgaaattt 480

atattataat aatgaaatga tataacttaa aagaagttgt ttgatatctt atcagaatca 540

tgcaggtact cataatataa gaaataatta tgatgaaatt tatatatgtt ttatgcagag 600

atttattacg cattgtttac ttgggttatg tattacttat ttcatctttt atcagaatgt 660

aaaattatca ttcaataaga aatccaattc tgttaaattc aaaatacaaa caataacatt 720

ttcaagaccg attttttgcc caagaatata cagtaaacat atttatgata tggtaggtct 780

ctttagtaat tgaccaacaa ggattgtggt ggagtgggaa atactcttta atacttcacc 840

aagaggtctc caatttgagc ccctgaatac gaaatcgtct ttgttagtat ataccctaac 900

ctaatacaaa aattagtata ttagccttna cagctaaaat ctttgtgacc tgtaagtcac 960

gcgaggacaa atttaccgta acaccaactt attcatgata taattgtccc ttttagcacg 1020

gtaataatga ggtgggtaga aatttattac ttgagggccc tttctacacc cacccttatt 1080

ctcttgcttc aattattgaa ttgaagaagt aatgaaaaaa cagactccat tggataaagg 1140

acagtttgca aacacagctg taacaattta gagcactagc aaaatagaga gagttttgag 1200

agaaattttt gtttgcaaat tactcttaac cttcagcagg taaaataaag ttcttaactg 1260

agactatttg aagatatatt ttgttaaaga atcattttgt gtgtttcctt gttttgcttt 1320

tgcagatttg agaa 1334

<210> 3
<211> 1797
<212> DNA
<213> Fusarium oxysporum

<400> 3
aaaaaagcag ctttaaaaaa agtactttkg aaaggkgctg aaacttattt tttgaaataa 60

gcagttatgt gtttggaawa aaagtgctga agttgctatg tcaaacatga aaagggraaa 120

aatggaagaa agagwtgtta gggttatgtc gtaatttgga gattgtataa aaatattaag 180

ggcaaaaaaa ataaaaatgt gtcaacttaa aacagcttat aagctaaaag ttaaaagctg 240

gggtagaggt gttttttttt tttttagctt ataagttgtt ttaagttgac cacattttta 300

tttttkttgc ccttaatatt tttatacaat ctcaaaatta cgacataacc ctaacatctt 360

tttctcccat tttttccttt tcacgtttga catagcaact tcagcacttt tatccaaaca 420

cataactgct tattttaaaa ataagtttca gcactttcaa aagtactttt ttaaagctgc 480

ttttattaag cccatccaaa cgggccctaa aattgctaat gtttgctctt tctattctca 540

aactccgtaa tatttaagaa aatttgctaa tgataggtca cttttaacac taaataatta 600

taaattgggt agaaatttat ttatcatttt aagctttttt aattttgagt cttctcccta 660

attaagaccc ttcccctctt gcttcaatta tttaactgaa tagtctttgt cttattgttg 720

ggtgaaagtc tgtcttcttg ttaggtacta agtcctacaa taatatcaat aatttgctat 780

ggagaaaaaa atattatagg agaaaaataa ttaattttaa ttcatgaata tgtcttaata 840

tgcaactcat tttgcttata tatatcaaat taaactctgt tcctttaact ttttcctatg 900

aagatacatt ttaatttatt tgatgaggtt agttttgaaa tttatattat aataatgaaa 960

tgatataact taaaagaagt tgtttgatat cttatcagaa tcatgcaggt actcataata 1020

taagaaataa ttatgatgaa atttatatat gttttatgca gagatttatt acgcattgtt 1080

tacttgggtt atgtattact tatttcatct tttatcagaa tgtaaaatta tcattcaata 1140

agaaatccaa ttctgttaaa ttcaaaatac aaacaataac attttcaaga ccgatttttt 1200

gcccaagaat atacagtaaa catatttatg atatggtagg tctctttagt aattgaccaa 1260

caaggattgt ggtggagtgg gaaatactct ttaatacttc accaagaggt ctccaatttg 1320

agcccctgaa tacgaaatcg tctttgttag tatataccct aacctaatac aaaaattagt 1380

atattagcct tnacagctaa aatctttgtg acctgtaagt cacgcgagga caaatttacc 1440

gtaacaccaa cttattcatg atataattgt cccttttagc acggtaataa tgaggtgggt 1500

agaaatttat tacttgaggg ccctttctac acccaccctt attctcttgc ttcaattatt 1560

gaattgaaga agtaatgaaa aaacagactc cattggataa aggacagttt gcaaacacag 1620

ctgtaacaat ttagagcact agcaaaatag agagagtttt gagagaaatt tttgtttgca 1680

aattactctt aaccttcagc aggtaaaata aagttcttaa ctgagactat ttgaagatat 1740

attttgttaa agaatcattt tgtgtgtttc cttgttttgc ttttgcagat ttgagaa 1797

<210> 4
<211> 3801
<212> DNA
<213> Fusarium oxysporum

<220>
<221> CDS
<222> (1)..(3801)
<223> nucleotide sequence of the I-2 resistance gene

<400> 4
atg gag att ggc tta gca gtt ggt ggt gca ttt ctc tcc tca gct ttg 48
Met Glu Ile Gly Leu Ala Val Gly Gly Ala Phe Leu Ser Ser Ala Leu
1               5                   10                  15

aat gtt ctg ttt gat agg ctt gct cct aac ggt gat ctg ctc aac atg 96
Asn Val Leu Phe Asp Arg Leu Ala Pro Asn Gly Asp Leu Leu Asn Met
            20                  25                  30

ttt cgg aag cat aag gat cat gtt aag ctc tta aag aag ctg aaa atg 144
Phe Arg Lys His Lys Asp His Val Lys Leu Leu Lys Lys Leu Lys Met
        35                  40                  45

act ttg cgt ggt att cag att gtg cta agt gat gca gag aat aag caa 192
Thr Leu Arg Gly Ile Gln Ile Val Leu Ser Asp Ala Glu Asn Lys Gln
    50                  55                  60

gca tca aat cca tct gtg aga gac tgg ctt aat gag ctt cga gat gct 240
Ala Ser Asn Pro Ser Val Arg Asp Trp Leu Asn Glu Leu Arg Asp Ala
65                  70                  75                  80

gtc gac tct gct gaa aat tta ata gaa gaa gtc aat tat gaa gct ttg 288
Val Asp Ser Ala Glu Asn Leu Ile Glu Glu Val Asn Tyr Glu Ala Leu
                85                  90                  95

agg ctt aag gtg gaa ggt cag cat cag aat ttt tca gaa aca agc aac 336
Arg Leu Lys Val Glu Gly Gln His Gln Asn Phe Ser Glu Thr Ser Asn
            100                 105                 110

cag caa gta agt gat gat ttt ttc ctt aac ata aag gac aag ctg gaa 384
Gln Gln Val Ser Asp Asp Phe Phe Leu Asn Ile Lys Asp Lys Leu Glu
        115                 120                 125

gac act att gaa aca tta aag gat ttg caa gag caa att ggt ctc ctt 432
Asp Thr Ile Glu Thr Leu Lys Asp Leu Gln Glu Gln Ile Gly Leu Leu
    130                 135                 140

ggc tta aag gag tat ttt gat tcc acg aaa cta gaa act aga aga cct     480
Gly Leu Lys Glu Tyr Phe Asp Ser Thr Lys Leu Glu Thr Arg Arg Pro
145                 150                 155                 160

tca act tct gtg gat gat gaa tct gat atc ttt ggt agg cag agc gaa     528
Ser Thr Ser Val Asp Asp Glu Ser Asp Ile Phe Gly Arg Gln Ser Glu
                165                 170                 175

ata gag gat ttg att gac cgt cta ttg tct gaa ggt gca agt ggg aaa     576
Ile Glu Asp Leu Ile Asp Arg Leu Leu Ser Glu Gly Ala Ser Gly Lys
            180                 185                 190

aag ctg aca gta gtt cct atc gtt gga atg ggc ggc cag ggc aag aca     624
Lys Leu Thr Val Val Pro Ile Val Gly Met Gly Gly Gln Gly Lys Thr
        195                 200                 205

aca ctt gct aaa gcc gta tac aat gat gag agg gtg aag aat cat ttt     672
Thr Leu Ala Lys Ala Val Tyr Asn Asp Glu Arg Val Lys Asn His Phe
    210                 215                 220

gat ttg aaa gcg tgg tat tgc gtt tct gaa gga ttt gat gct ttg aga     720
Asp Leu Lys Ala Trp Tyr Cys Val Ser Glu Gly Phe Asp Ala Leu Arg
225                 230                 235                 240

ata aca aaa gaa tta ctc caa gaa att ggc aaa ttt gac tcg aag gat     768
Ile Thr Lys Glu Leu Leu Gln Glu Ile Gly Lys Phe Asp Ser Lys Asp
                245                 250                 255

gtc cac aac aat ctt aac cag ctt caa gtc aaa ttg aag gaa agt ttg     816
Val His Asn Asn Leu Asn Gln Leu Gln Val Lys Leu Lys Glu Ser Leu
            260                 265                 270

aag gga aag aag ttc ctt att gtt ttg gat gat gtg tgg aat gaa aat     864
Lys Gly Lys Lys Phe Leu Ile Val Leu Asp Asp Val Trp Asn Glu Asn
        275                 280                 285

tac aac gag tgg aat gac ttg aga aat att ttt gca caa gga gat ata     912
Tyr Asn Glu Trp Asn Asp Leu Arg Asn Ile Phe Ala Gln Gly Asp Ile
    290                 295                 300

gga agt aag atc att gtg acg aca cgc aaa gac agt gtt gcc ttg atg     960
Gly Ser Lys Ile Ile Val Thr Thr Arg Lys Asp Ser Val Ala Leu Met
305                 310                 315                 320

atg gga aat gag caa att cgc atg ggc aat ttg tct acc gaa gcc tct     1008
Met Gly Asn Glu Gln Ile Arg Met Gly Asn Leu Ser Thr Glu Ala Ser
                325                 330                 335

tgg tct tta ttt caa aga cat gca ttt gaa aac atg gat cct atg gga     1056
Trp Ser Leu Phe Gln Arg His Ala Phe Glu Asn Met Asp Pro Met Gly
            340                 345                 350

cat ccg gaa ctt gaa gag gtc gga aga caa att gca gcc aag tgc aaa 1104
His Pro Glu Leu Glu Glu Val Gly Arg Gln Ile Ala Ala Lys Cys Lys
        355                 360                 365

gga ctg ccc tta gct ctg aag acg ctc gct ggc atg tta cgc tcc aaa 1152
Gly Leu Pro Leu Ala Leu Lys Thr Leu Ala Gly Met Leu Arg Ser Lys
    370                 375                 380

tca gag gtt gaa gag tgg aaa cgt att ttg aga agt gaa ata tgg gag 1200
Ser Glu Val Glu Glu Trp Lys Arg Ile Leu Arg Ser Glu Ile Trp Glu
385                 390                 395                 400

ctg cca cac aat gac ata tta cca gcg ttg atg ttg agc tac aat gat 1248
Leu Pro His Asn Asp Ile Leu Pro Ala Leu Met Leu Ser Tyr Asn Asp
                405                 410                 415

ctt ccc gca cat tta aag cga tgc ttt tct ttt tgt gca ata ttt cct 1296
Leu Pro Ala His Leu Lys Arg Cys Phe Ser Phe Cys Ala Ile Phe Pro
            420                 425                 430

aaa gat tat cca ttt agg aaa gaa caa gtt att cat cta tgg att gcc 1344
Lys Asp Tyr Pro Phe Arg Lys Glu Gln Val Ile His Leu Trp Ile Ala
        435                 440                 445

aat ggt ctc gta cca gtg aaa gat gaa ata aat caa gat tta ggc aac 1392
Asn Gly Leu Val Pro Val Lys Asp Glu Ile Asn Gln Asp Leu Gly Asn
    450                 455                 460

caa tac ttt cta gag ttg aga tca aga tca tta ttt gaa aag gtc cca 1440
Gln Tyr Phe Leu Glu Leu Arg Ser Arg Ser Leu Phe Glu Lys Val Pro
465                 470                 475                 480

aat cct tct aaa agg aac ata gag gaa tta ttc ctt atg cat gac ctt 1488
Asn Pro Ser Lys Arg Asn Ile Glu Glu Leu Phe Leu Met His Asp Leu
                485                 490                 495

gtc aat gat tta gcc caa ctt gca tct tca aaa ctt tgt atc agg tta 1536
Val Asn Asp Leu Ala Gln Leu Ala Ser Ser Lys Leu Cys Ile Arg Leu
            500                 505                 510

gaa gag agc caa gga tct cat atg ttg gaa caa tgt cgg cac tta tct 1584
Glu Glu Ser Gln Gly Ser His Met Leu Glu Gln Cys Arg His Leu Ser
        515                 520                 525

tat tca ata gga ttt aat ggt gag ttt aag aaa ttg aca ccc ctc tac 1632
Tyr Ser Ile Gly Phe Asn Gly Glu Phe Lys Lys Leu Thr Pro Leu Tyr
    530                 535                 540

aaa ttg gag cag ttg agg aca ttg ctt ccg ata cgt att gaa ttc aga 1680
Lys Leu Glu Gln Leu Arg Thr Leu Leu Pro Ile Arg Ile Glu Phe Arg
545                 550                 555                 560

ttg cac aat cta agc aag agg gtg ttg cat aac ata ctg cct aca cta 1728
Leu His Asn Leu Ser Lys Arg Val Leu His Asn Ile Leu Pro Thr Leu
                565                 570                 575

aga tcc ttg agg gcc cta tca ttc tct caa tac aag att aag gag ttg 1776
Arg Ser Leu Arg Ala Leu Ser Phe Ser Gln Tyr Lys Ile Lys Glu Leu
            580                 585                 590

cca aat gac ttg ttt acc aaa tta aag ctc ctc aga ttt ttg gat att 1824
Pro Asn Asp Leu Phe Thr Lys Leu Lys Leu Leu Arg Phe Leu Asp Ile
        595                 600                 605

tct cgg aca tgg att aca aag ttg ccg gat tcc att tgt gga tta tat 1872
Ser Arg Thr Trp Ile Thr Lys Leu Pro Asp Ser Ile Cys Gly Leu Tyr
    610                 615                 620

aac ttg gag aca ctt ctc ctg tca tct tgt gct gat ctt gag gag cta 1920
Asn Leu Glu Thr Leu Leu Leu Ser Ser Cys Ala Asp Leu Glu Glu Leu
625                 630                 635                 640

ccg ctg cag atg gag aag ttg att aac ttg cgt cat ctt gac gta agc 1968
Pro Leu Gln Met Glu Lys Leu Ile Asn Leu Arg His Leu Asp Val Ser
                645                 650                 655

aac act cgg cgc ttg aag atg cca cta cat ctg agc agg ttg aaa agc 2016
Asn Thr Arg Arg Leu Lys Met Pro Leu His Leu Ser Arg Leu Lys Ser
            660                 665                 670

ctc caa gtg ttg gtg gga ccc aag ttt ttt gta gat ggt tgg aga atg 2064
Leu Gln Val Leu Val Gly Pro Lys Phe Phe Val Asp Gly Trp Arg Met
        675                 680                 685

gaa gat ttg ggt gaa gca caa aac tta cat gga tct cta tca gtt gtg 2112
Glu Asp Leu Gly Glu Ala Gln Asn Leu His Gly Ser Leu Ser Val Val
    690                 695                 700

aag ttg gaa aat gtg gtt gat aga agg gaa gct gtg aag gca aag atg 2160
Lys Leu Glu Asn Val Val Asp Arg Arg Glu Ala Val Lys Ala Lys Met
705                 710                 715                 720

agg gag aag aat cat gtt gag caa tta tca ttg gag tgg agt gaa agt 2208
Arg Glu Lys Asn His Val Glu Gln Leu Ser Leu Glu Trp Ser Glu Ser
                725                 730                 735

agt att gcc gac aat tca caa aca gaa agt gac ata ctt gat gag cta 2256
Ser Ile Ala Asp Asn Ser Gln Thr Glu Ser Asp Ile Leu Asp Glu Leu
            740                 745                 750

tgc cca cat aaa aac atc aaa aaa gtc gaa atc agt gga tat aga ggg 2304
Cys Pro His Lys Asn Ile Lys Lys Val Glu Ile Ser Gly Tyr Arg Gly
        755                 760                 765

aca aac ttt ccc aat tgg gta gct gat cct ttg ttt ctt aag ctg gtg     2352
Thr Asn Phe Pro Asn Trp Val Ala Asp Pro Leu Phe Leu Lys Leu Val
    770                 775                 780

aat ttg tct cta aga aac tgc aag gac tgt tac tcc ttg cca gca cta     2400
Asn Leu Ser Leu Arg Asn Cys Lys Asp Cys Tyr Ser Leu Pro Ala Leu
785                 790                 795                 800

gga caa ctc cct tgt ttg aaa ttc ctt tcc gtt aaa ggg atg cat gga     2448
Gly Gln Leu Pro Cys Leu Lys Phe Leu Ser Val Lys Gly Met His Gly
                805                 810                 815

ata aga gtg gtg acg gaa gaa ttc tat ggc aga ttg tcc tcc aaa aag     2496
Ile Arg Val Val Thr Glu Glu Phe Tyr Gly Arg Leu Ser Ser Lys Lys
            820                 825                 830

cct ttt aac tct cta gag aag ctt gaa ttt gaa gat atg acg gag tgg     2544
Pro Phe Asn Ser Leu Glu Lys Leu Glu Phe Glu Asp Met Thr Glu Trp
        835                 840                 845

aag caa tgg cac gca cta gga att gga gag ttc cct aca ctt gag aac     2592
Lys Gln Trp His Ala Leu Gly Ile Gly Glu Phe Pro Thr Leu Glu Asn
    850                 855                 860

ctt tca att aaa aat tgc cct gag ctc agt ttg gag ata ccc atc caa     2640
Leu Ser Ile Lys Asn Cys Pro Glu Leu Ser Leu Glu Ile Pro Ile Gln
865                 870                 875                 880

ttt tca agt tta aaa agg tta gaa gtt agt gat tgt cca gtt gtt ttt     2688
Phe Ser Ser Leu Lys Arg Leu Glu Val Ser Asp Cys Pro Val Val Phe
                885                 890                 895

gat gat gcc caa ctg ttt aga tcc caa ctt gag gca atg aag cag att     2736
Asp Asp Ala Gln Leu Phe Arg Ser Gln Leu Glu Ala Met Lys Gln Ile
            900                 905                 910

gag gaa ata gat ata tgt gat tgt aac tct gtt acc tcc ttt cct ttt     2784
Glu Glu Ile Asp Ile Cys Asp Cys Asn Ser Val Thr Ser Phe Pro Phe
        915                 920                 925

agc ata ctg cca act acc ttg aag aga ata cag ata tct cgt tgc cca     2832
Ser Ile Leu Pro Thr Thr Leu Lys Arg Ile Gln Ile Ser Arg Cys Pro
    930                 935                 940

aaa ttg aaa ttg gag gcg cca gtt ggt gag atg ttt gtg gag tat ttg     2880
Lys Leu Lys Leu Glu Ala Pro Val Gly Glu Met Phe Val Glu Tyr Leu
945                 950                 955                 960

aga gtg aat gat tgt ggt tgt gta gat gat ata tca cct gag ttt ctc     2928
Arg Val Asn Asp Cys Gly Cys Val Asp Asp Ile Ser Pro Glu Phe Leu
                965                 970                 975

cca aca gca cgt caa ttg agt att gaa aat tgc cag aac gtt act agg 2976
Pro Thr Ala Arg Gln Leu Ser Ile Glu Asn Cys Gln Asn Val Thr Arg
            980                 985                 990

ttt ttg att cct act gcc act gaa act ctc cgt att tcg aat tgt gag 3024
Phe Leu Ile Pro Thr Ala Thr Glu Thr Leu Arg Ile Ser Asn Cys Glu
        995                 1000                1005

aat gtt gaa aaa cta tcg gtg gca tgt gga gga gcg gcc cag atg acg 3072
Asn Val Glu Lys Leu Ser Val Ala Cys Gly Gly Ala Ala Gln Met Thr
    1010                1015                1020

tca ctg aat att tgg gga tgt aag aag ctc aag tgt ctt cca gaa ctc 3120
Ser Leu Asn Ile Trp Gly Cys Lys Lys Leu Lys Cys Leu Pro Glu Leu
1025                1030                1035                1040

ctt cca tct ctc aag gaa ctg cgt ctg tct gat tgt cca gaa ata gaa 3168
Leu Pro Ser Leu Lys Glu Leu Arg Leu Ser Asp Cys Pro Glu Ile Glu
                1045                1050                1055

gga gaa ttg ccc ttc aat tta gaa ata ctc cgt atc ata tat tgc aag 3216
Gly Glu Leu Pro Phe Asn Leu Glu Ile Leu Arg Ile Ile Tyr Cys Lys
            1060                1065                1070

aaa ctg gtg aat ggc cga aag gag tgg cat tta cag aga ctc aca gag 3264
Lys Leu Val Asn Gly Arg Lys Glu Trp His Leu Gln Arg Leu Thr Glu
        1075                1080                1085

tta tgg atc gat cat gat ggg agt gac gaa gat att gaa cat tgg gag 3312
Leu Trp Ile Asp His Asp Gly Ser Asp Glu Asp Ile Glu His Trp Glu
    1090                1095                1100

ttg cct tgt tct att cag aga ctt acc ata aag aat ctt aaa aca tta 3360
Leu Pro Cys Ser Ile Gln Arg Leu Thr Ile Lys Asn Leu Lys Thr Leu
1105                1110                1115                1120

agc agc caa cat ctc aaa agc ctc acc tct ctt caa tat cta tgt att 3408
Ser Ser Gln His Leu Lys Ser Leu Thr Ser Leu Gln Tyr Leu Cys Ile
                1125                1130                1135

gag ggt tat tta tct cag att cag tca caa ggc cag ctt tcc tcc ttt 3456
Glu Gly Tyr Leu Ser Gln Ile Gln Ser Gln Gly Gln Leu Ser Ser Phe
            1140                1145                1150

tct cac ctc act tcg ctt caa act cta caa atc tgg aat ttc ctt aat 3504
Ser His Leu Thr Ser Leu Gln Thr Leu Gln Ile Trp Asn Phe Leu Asn
        1155                1160                1165

ctc caa tca ctt gct gaa tca gca ctg ccc tcc tcc ctc tct cac ctg 3552
Leu Gln Ser Leu Ala Glu Ser Ala Leu Pro Ser Ser Leu Ser His Leu
    1170                1175                1180

gag ata gat gat tgc cct aat ctc caa tca ctc ttc gaa tca gca ctg     3600
Glu Ile Asp Asp Cys Pro Asn Leu Gln Ser Leu Phe Glu Ser Ala Leu
1185                1190                1195                1200

ccc tcc tcc ctc tct cag ctg ttc atc cag gat tgc cct aat ctc caa     3648
Pro Ser Ser Leu Ser Gln Leu Phe Ile Gln Asp Cys Pro Asn Leu Gln
                1205                1210                1215

tcc ctt cca ttt aaa ggg atg ccc tct tcc ctc tct aaa cta tct att     3696
Ser Leu Pro Phe Lys Gly Met Pro Ser Ser Leu Ser Lys Leu Ser Ile
            1220                1225                1230

ttc aat tgc cca ttg ctc aca cca cta cta gaa ttt gac aag ggg gaa     3744
Phe Asn Cys Pro Leu Leu Thr Pro Leu Leu Glu Phe Asp Lys Gly Glu
        1235                1240                1245

tac tgg cca caa att gct cat att ccc atc ata aat atc gat tgg aaa     3792
Tyr Trp Pro Gln Ile Ala His Ile Pro Ile Ile Asn Ile Asp Trp Lys
    1250                1255                1260

tat att taa                                                         3801
Tyr Ile
1265

<210> 5
<211> 1267
<212> PRT
<213> Fusarium oxysporum

<400> 5
Met Glu Ile Gly Leu Ala Val Gly Gly Ala Phe Leu Ser Ser Ala Leu
1               5                   10                  15

Asn Val Leu Phe Asp Arg Leu Ala Pro Asn Gly Asp Leu Leu Asn Met
            20                  25                  30

Phe Arg Lys His Lys Asp His Val Lys Leu Leu Lys Lys Leu Lys Met
        35                  40                  45

Thr Leu Arg Gly Ile Gln Ile Val Leu Ser Asp Ala Glu Asn Lys Gln
    50                  55                  60

Ala Ser Asn Pro Ser Val Arg Asp Trp Leu Asn Glu Leu Arg Asp Ala
65                  70                  75                  80

Val Asp Ser Ala Glu Asn Leu Ile Glu Glu Val Asn Tyr Glu Ala Leu
                85                  90                  95

Arg Leu Lys Val Glu Gly Gln His Gln Asn Phe Ser Glu Thr Ser Asn
            100                 105                 110

Gln Gln Val Ser Asp Asp Phe Phe Leu Asn Ile Lys Asp Lys Leu Glu
        115                 120                 125

Asp Thr Ile Glu Thr Leu Lys Asp Leu Gln Glu Gln Ile Gly Leu Leu
    130                 135                 140

Gly Leu Lys Glu Tyr Phe Asp Ser Thr Lys Leu Glu Thr Arg Arg Pro
145                 150                 155                 160

Ser Thr Ser Val Asp Asp Glu Ser Asp Ile Phe Gly Arg Gln Ser Glu
                165                 170                 175

Ile Glu Asp Leu Ile Asp Arg Leu Leu Ser Glu Gly Ala Ser Gly Lys
            180                 185                 190

Lys Leu Thr Val Val Pro Ile Val Gly Met Gly Gly Gln Gly Lys Thr
        195                 200                 205

Thr Leu Ala Lys Ala Val Tyr Asn Asp Glu Arg Val Lys Asn His Phe
    210                 215                 220

Asp Leu Lys Ala Trp Tyr Cys Val Ser Glu Gly Phe Asp Ala Leu Arg
225                 230                 235                 240

Ile Thr Lys Glu Leu Leu Gln Glu Ile Gly Lys Phe Asp Ser Lys Asp
                245                 250                 255

Val His Asn Asn Leu Asn Gln Leu Gln Val Lys Leu Lys Glu Ser Leu
            260                 265                 270

Lys Gly Lys Lys Phe Leu Ile Val Leu Asp Asp Val Trp Asn Glu Asn
        275                 280                 285

Tyr Asn Glu Trp Asn Asp Leu Arg Asn Ile Phe Ala Gln Gly Asp Ile
    290                 295                 300

Gly Ser Lys Ile Ile Val Thr Thr Arg Lys Asp Ser Val Ala Leu Met
305                 310                 315                 320

Met Gly Asn Glu Gln Ile Arg Met Gly Asn Leu Ser Thr Glu Ala Ser
                325                 330                 335

Trp Ser Leu Phe Gln Arg His Ala Phe Glu Asn Met Asp Pro Met Gly
            340                 345                 350

His Pro Glu Leu Glu Glu Val Gly Arg Gln Ile Ala Ala Lys Cys Lys
        355                 360                 365

Gly Leu Pro Leu Ala Leu Lys Thr Leu Ala Gly Met Leu Arg Ser Lys
    370                 375                 380

Ser Glu Val Glu Glu Trp Lys Arg Ile Leu Arg Ser Glu Ile Trp Glu
385                 390                 395                 400

Leu Pro His Asn Asp Ile Leu Pro Ala Leu Met Leu Ser Tyr Asn Asp
                405                 410                 415

Leu Pro Ala His Leu Lys Arg Cys Phe Ser Phe Cys Ala Ile Phe Pro
            420                 425                 430

Lys Asp Tyr Pro Phe Arg Lys Glu Gln Val Ile His Leu Trp Ile Ala
        435                 440                 445

Asn Gly Leu Val Pro Val Lys Asp Glu Ile Asn Gln Asp Leu Gly Asn
    450                 455                 460

Gln Tyr Phe Leu Glu Leu Arg Ser Arg Ser Leu Phe Glu Lys Val Pro
465                 470                 475                 480

Asn Pro Ser Lys Arg Asn Ile Glu Glu Leu Phe Leu Met His Asp Leu
                485                 490                 495

Val Asn Asp Leu Ala Gln Leu Ala Ser Ser Lys Leu Cys Ile Arg Leu
            500                 505                 510

Glu Glu Ser Gln Gly Ser His Met Leu Glu Gln Cys Arg His Leu Ser
        515                 520                 525

Tyr Ser Ile Gly Phe Asn Gly Glu Phe Lys Lys Leu Thr Pro Leu Tyr
    530                 535                 540

Lys Leu Glu Gln Leu Arg Thr Leu Leu Pro Ile Arg Ile Glu Phe Arg
545                 550                 555                 560

Leu His Asn Leu Ser Lys Arg Val Leu His Asn Ile Leu Pro Thr Leu
                565                 570                 575

Arg Ser Leu Arg Ala Leu Ser Phe Ser Gln Tyr Lys Ile Lys Glu Leu
            580                 585                 590

Pro Asn Asp Leu Phe Thr Lys Leu Lys Leu Leu Arg Phe Leu Asp Ile
        595                 600                 605

Ser Arg Thr Trp Ile Thr Lys Leu Pro Asp Ser Ile Cys Gly Leu Tyr
    610                 615                 620

Asn Leu Glu Thr Leu Leu Leu Ser Ser Cys Ala Asp Leu Glu Glu Leu
625                 630                 635                 640

Pro Leu Gln Met Glu Lys Leu Ile Asn Leu Arg His Leu Asp Val Ser
                645                 650                 655

Asn Thr Arg Arg Leu Lys Met Pro Leu His Leu Ser Arg Leu Lys Ser
            660                 665                 670

Leu Gln Val Leu Val Gly Pro Lys Phe Phe Val Asp Gly Trp Arg Met
        675                 680                 685

Glu Asp Leu Gly Glu Ala Gln Asn Leu His Gly Ser Leu Ser Val Val
    690                 695                 700

Lys Leu Glu Asn Val Val Asp Arg Arg Glu Ala Val Lys Ala Lys Met
705                 710                 715                 720

Arg Glu Lys Asn His Val Glu Gln Leu Ser Leu Glu Trp Ser Glu Ser
                725                 730                 735

Ser Ile Ala Asp Asn Ser Gln Thr Glu Ser Asp Ile Leu Asp Glu Leu
            740                 745                 750

Cys Pro His Lys Asn Ile Lys Lys Val Glu Ile Ser Gly Tyr Arg Gly
        755                 760                 765

Thr Asn Phe Pro Asn Trp Val Ala Asp Pro Leu Phe Leu Lys Leu Val
    770                 775                 780

Asn Leu Ser Leu Arg Asn Cys Lys Asp Cys Tyr Ser Leu Pro Ala Leu
785                 790                 795                 800

Gly Gln Leu Pro Cys Leu Lys Phe Leu Ser Val Lys Gly Met His Gly
                805                 810                 815

Ile Arg Val Val Thr Glu Glu Phe Tyr Gly Arg Leu Ser Ser Lys Lys
            820                 825                 830

Pro Phe Asn Ser Leu Glu Lys Leu Glu Phe Glu Asp Met Thr Glu Trp
        835                 840                 845

Lys Gln Trp His Ala Leu Gly Ile Gly Glu Phe Pro Thr Leu Glu Asn
    850                 855                 860

Leu Ser Ile Lys Asn Cys Pro Glu Leu Ser Leu Glu Ile Pro Ile Gln
865                 870                 875                 880

Phe Ser Ser Leu Lys Arg Leu Glu Val Ser Asp Cys Pro Val Val Phe
                885                 890                 895

Asp Asp Ala Gln Leu Phe Arg Ser Gln Leu Glu Ala Met Lys Gln Ile
            900                 905                 910

Glu Glu Ile Asp Ile Cys Asp Cys Asn Ser Val Thr Ser Phe Pro Phe
        915                 920                 925

Ser Ile Leu Pro Thr Thr Leu Lys Arg Ile Gln Ile Ser Arg Cys Pro
    930                 935                 940

Lys Leu Lys Leu Glu Ala Pro Val Gly Glu Met Phe Val Glu Tyr Leu
945                 950                 955                 960

Arg Val Asn Asp Cys Gly Cys Val Asp Asp Ile Ser Pro Glu Phe Leu
                965                 970                 975

Pro Thr Ala Arg Gln Leu Ser Ile Glu Asn Cys Gln Asn Val Thr Arg
            980                 985                 990

Phe Leu Ile Pro Thr Ala Thr Glu Thr Leu Arg Ile Ser Asn Cys Glu
        995                 1000                1005

Asn Val Glu Lys Leu Ser Val Ala Cys Gly Gly Ala Ala Gln Met Thr
    1010                1015                1020

Ser Leu Asn Ile Trp Gly Cys Lys Lys Leu Lys Cys Leu Pro Glu Leu
025 1030 1035 1040

Leu Pro Ser Leu Lys Glu Leu Arg Leu Ser Asp Cys Pro Glu Ile Glu
                1045                1050                1055

Gly Glu Leu Pro Phe Asn Leu Glu Ile Leu Arg Ile Ile Tyr Cys Lys
            1060                1065                1070

Lys Leu Val Asn Gly Arg Lys Glu Trp His Leu Gln Arg Leu Thr Glu
        1075                1080                1085

Leu Trp Ile Asp His Asp Gly Ser Asp Glu Asp Ile Glu His Trp Glu
    1090                1095                1100

Leu Pro Cys Ser Ile Gln Arg Leu Thr Ile Lys Asn Leu Lys Thr Leu
105 1110 1115 1120

Ser Ser Gln His Leu Lys Ser Leu Thr Ser Leu Gln Tyr Leu Cys Ile
                1125                1130                1135

Glu Gly Tyr Leu Ser Gln Ile Gln Ser Gln Gly Gln Leu Ser Ser Phe
            1140                1145                1150

Ser His Leu Thr Ser Leu Gln Thr Leu Gln Ile Trp Asn Phe Leu Asn
        1155                1160                1165

Leu Gln Ser Leu Ala Glu Ser Ala Leu Pro Ser Ser Leu Ser His Leu
    1170                1175                1180

Glu Ile Asp Asp Cys Pro Asn Leu Gln Ser Leu Phe Glu Ser Ala Leu
185 1190 1195 1200

Pro Ser Ser Leu Ser Gln Leu Phe Ile Gln Asp Cys Pro Asn Leu Gln
                1205                1210                1215

Ser Leu Pro Phe Lys Gly Met Pro Ser Ser Leu Ser Lys Leu Ser Ile
            1220                1225                1230

Phe Asn Cys Pro Leu Leu Thr Pro Leu Leu Glu Phe Asp Lys Gly Glu
        1235                1240                1245

Tyr Trp Pro Gln Ile Ala His Ile Pro Ile Ile Asn Ile Asp Trp Lys
    1250                1255                1260

Tyr Ile
1265

## Description

The invention relates to nucleotide sequences having a tissue specific regulatory activity on the transcription of genes in plants.

The invention is also directed to nucleotide sequences and especially DNA sequences including chimeric or recombinant constructs, wherein nucleotide sequences having a regulatory activity are used to regulate especially to initiate the transcription of nucleic acid sequences of interest in determined host cells, especially plant cells. These regulatory sequences are sometimes designated as promoter fragments, a broad meaning being given to the term "promoter".

In prior international patent application WO 97/06259, the inventors have described the isolation characterization and sequencing of a nucleic acid sequence comprising the I-2 resistance gene, which gene is involved in the resistance mechanism of plants, to plant pathogens especially to fungi and more particularly to soilborne fungi of the *Fusarium* species.

Among the pathogens causing serious damages to plants, one finds the group of soilborne cortical rots and vascular wilt inducing fungi, such as *Fusarium* and *Verticillium* (Toussoun, 1981, in: Fusarium Diseases, Biology and Taxonomy, Nelson, Toussoun & Cook edit., Penn. State Univ. Press, 457 pp.). Said wilt inducing fungi infect the plants through the roots via direct penetration or via wounds after which the xylem vascular tissue of the plant is colonized and symptoms of infection with said fungi are wilting, browning and dying of leaves followed by plant death. Entire plants or plant parts above the point of vascular invasion of the pathogen may die within a period of some weeks after infection. The fungi usually spread internally through the xylem vessels as mycelium or conidia until the entire plant is killed. Because of the fact that those fungi are able to survive in the soil saprophytically, they become established forever once they are introduced in the field. They are distributed more or less worldwide causing tremendous losses on most species of vegetables and flowers, field crops, fruit trees, etc. Because those fungi are so widespread and so persistent in soils the only effective way of controlling said wilt inducing fungi is using resistant plant genotypes.

Most Fusarium species belong to the family of imperfect fungi. This class of fungi is characterized by the fact that only a vegetative stage of the fungus is known. The generative stage of those fungi has not been discovered yet. Due to the overall classification or taxonomy of fungi upon their morphological characteristics of the generative phase, one should bear in mind that fungi belonging to the class of imperfect fungi can be classified into another class once their generative stage is discovered and, subsequently, a change of classification and name can follow (Gerlach, 1981, in: Fusarium, Diseases, Biology and Taxonomy, Nelson, Toussoun & Cook edit., Penn. State Univ. Press, p. 413-426). Moreover, it has been observed that some Fusarium species can "mutate" into another species depending on the plant infected and/or the environment (Bolton & Davidson, 1972, Can. J. Plant Sci, **52**, 189-196). Up to now most of the wilt inducing Fusarium belong to the species *Fusarium oxysporum.* Different plant species are attacked by different races or isolates of Fusarium (Armstrong & Armstrong, 1981, in: Fusarium, Diseases, Biology and Taxonomy, Nelson, Toussoun & Cook edit., Penn. State Univ. Press, p. 391-399). However, some Fusarium isolates can infect different plant species. Known Fusarium isolates are for example: *Fusarium oxysporum* f.sp. *lycopersici* (tomato), including *Fusarium oxysporum* f.sp. licopersici race 2, *F. oxysporum* f.sp. *melonis* (melon), *F. oxysporum* f.sp. *batatas* (sweet potato, tobacco), *F. oxysporum* f.sp. *cepae* (onion), *F. oxysporum* f.sp. *conglutinans* (cabbage, radish), *F. oxysporum* f.sp. *cubense* (banana), *F. oxysporum* f.sp. *vasinfectum* (cotton, alfalfa, soybean, tobacco) *F. oxysporum* f.sp. *dianthii* (carnation), *F. oxysporum* f.sp. *chrysanthemi* (chrysanthemum), *F. oxysporum* f.sp. *tuberosi* (potato), F. *oxysporum* f.sp. *cyclaminis* (cyclamen), *F. oxysporum* f.sp. *nicotianae* (tobacco).

Therefore, the name of the wilt inducing fungi can change in the future.

The characterization of the I-2 resistance gene according to WO 97/06259 has enabled the inventors to identify the gene product encoded by this I-2 resistance gene, as a polypeptide which is capable of conferring to a plant susceptible to wilt inducing fungi, resistance to infection by said pathogen(s) and/or resistance to the disease induced by said pathogen, after said plant has been transformed with the I-2 gene. The nature of resistance conferred can vary upon different factors and can encompass preventing the infection by the plant pathogen and/or as example using a signal from the invading pathogen for the activation of a defense mechanism by a related gene.

In prior international patent application, several cosmid clones including cosmid designated under B22 and A55 have been described and it has been observed that the DNA segment containing the I-2 resistance gene would encompass a region of approximately 8 kb in size. The B22 cosmid having a 17 kb size which contains said gene under a functional form, has been deposited on July 14, 1995 as plasmid pKGI2-B22 at Centraal Bureau voor Schimmelcultures at Baarn (The Netherlands) under No. CBS546.95. The A55 cosmid was deposited at the same Collection on August 5, 1996 as plasmid pKGI2-A55 under No. CBS 820.96.

In addition, in said prior international patent application, the inventors have identified that a 1.3 kb DNA non coding fragment, upstream from the I-2 translation initiation codon (ATG) located at position 1 on SEQ ID NO:4 would be sufficient to drive the transcription of the I-2 resistance gene in plants transformed with said gene. By "sufficient", it is meant that transcripts are obtained when the coding sequence is placed under the control of said 1.3 kb fragment.

The 1.3 kb fragment is identified as a 1333 pb fragment located upstream from the ATG site of the coding sequence identified SEQ ID NO: 4 and corresponds to a fragment encompassing nucleotide 464 to nucleotide 1797 of the sequence identified under SEQ ID NO: 3.

Some results have been disclosed in WO 97/06259 showing that plants susceptible to wilt inducing fungi of Fusarium species, especially Fusarium *oxysporum f.sp lycopersici race 2,* are rendered resistant to said Fusarium when the coding sequence of the I-2 genes is transferred in said plants under the control of the above 1.3 kb fragment.

In order to examine the conditions and level of transcription of the I-2 resistance gene when placed under the control of a DNA sequence encompassing the above-said 1.3 kb fragment, and especially in view of the experimental results obtained that show a low abundancy of transcripts of the I-2 gene in some plant tissues, for instance in root, stem and leaf tissues of recombinant plants, the inventors have decided to further investigate the functionality of the I-2 5' coding sequences showing a regulatory activity on the transcription of the I-2 gene, and have therefore designed and tested recombinant constructs using a reporter gene placed under the control of nucleotide sequences comprising a regulatory sequence active on regulation of the transcription of the I-2 gene.

From the results obtained with DNA constructs using the regulatory transcription sequences of the I-2 gene, further compared with results obtained with the same reporter gene placed under the control of a different plant specific promoter, the inventors have characterized specific properties of the sequences disclosed in the present patent application, having a regulatory transcription activity: these properties include ability to enable a tissue specific expression of determined sequences placed under the control of these regulatory sequences.

Therefore, the invention provides means which can be used for regulation of the tissue specific expression in plant cells or plants, of nucleic acid sequences, especially as a result of regulation of the transcription of said nucleic acid sequences.

The invention therefore relates to a nucleotide sequence having a regulatory activity on the transcription of the I-2 resistance gene in determined plant host cells, which nucleotide sequence comprises a DNA sequence of around 3.8 kb derived from the 5' non coding region of the I-2 gene. Said sequence has preferably a size in the range of 5 to 1 kb.

According to a preferred embodiment, said regulatory sequence comprises the sequence depicted as SEQ ID NO:1, corresponding to a 3.8 kb fragment that can be obtained or derived from the 5' non coding region located upstream from the translation initiation codon (ATG) of said I-2 resistance gene. The coding sequence of the I-2 gene is represented as SEQ ID NO: 4.

The invention relates especially to said 3.8 kb fragment which can be obtained from the B22 cosmid and corresponds to an EcoRI/EcoNI fragment of 3.8 kb of the 5' end of the I-2 gene.

The invention especially relates to a nucleotide sequence according to the above definition, which sequence is SEQ ID NO: 1.

The above-identified sequence SEQ ID NO: 1 comprises a fragment located 5' from the ATG codon of the I-2 resistance gene which is sufficient for the transcription of said gene.

The invention also relates to the use of the 1.3 kb fragment derived from the 5' non coding region of the I-2 gene, said sequence being disclosed as SEQ ID NO:2 and containing nucleotide 464 to 1797 of the I-2 gene. It also concerns the use of the sequence designated SEQ ID NO:3 corresponding to nucleotides 1 to 1798 upstream from the ATG codon in the I-2 gene.

According to a particular embodiment, the invention also relates to DNA sequences retaining a regulatory activity of the sequences described above, especially of SEQ ID NO: 1, SEQ ID NO:2 or SEQ ID NO:3 which sequences are derived from the above by deletion, insertion, substitution of one or several nucleotide residues.

Despite their ability to regulate transcription of nucleic acid sequences, the choice of a specific sequence among the preceding one can lead to a modification of the level of the transcription.

Among derived regulatory nucleotide sequences used in accordance with the invention, those hybridizing under stringent conditions with one of the above described sequences are of interest to achieve the invention. Advantageously, said derived sequences have substantially the same size or a shorter size with respect to the sequence from which they are derived.

Stringent conditions refer to hybridization conditions which allow a nucleic acid sequence to hybridize to a particular sequence. In general, high stringent conditions refer to the hybridization conditions which allow a nucleic acid sequence of at least 50 nucleotides and preferably about 200 or more nucleotides to hybridize to a particular sequence at about 65 °C in a solution comprising about 1 M salt, preferably 6 x SSC or any other solution having a comparable ionic strength, and washing at 65 °C in a solution comprising about 0,1 M salt, or less, preferably 0,2 x SSC or any other solution having a comparable ionic strength. These conditions allow the detection of sequences having about 90 % or more sequence identity. In general, lower stringent conditions refer to the hybridization conditions which allow a nucleic acid sequence of at least 50 nucleotides and preferably about 200 or more nucleotides to hybridize to a particular sequence at about 45 °C in a solution comprising about 1 M salt, preferably 6 x SSC or any other solution having a comparable ionic strength, and washing at room temperature in a solution comprising about 1 M salt, preferably 6 x SSC or any other solution having a comparable ionic strength. These conditions allow the detection of sequences having up to 50 % sequence identity. The person skilled in the art will be able to modify these hybridization conditions in order to identify sequences varying in identity between 50 % and 90 %.

Alternatively, stringent conditions refer to hybridization conditions which allow a nucleic acid sequence to hybridize selectively to the *I-2* resistance gene in its genomic environment, substantially to the exclusion of hybridization with other DNA sequences of said genomic environment.

According to a preferred embodiment, the invention concerns regulatory nucleotide sequences according to the above definition or a fragment of said sequences which are capable of driving a tissue specific transcription of a determined nucleic acid sequence placed under their control.

By the expression "driving a tissue specific transcription", it is encompassed any sequence which would impact on the regulation of said transcription in a host cell and especially in a tissue specific manner, especially those capable of initiating said transcription.

The invention also relates to a recombinant nucleotide sequence comprising one of the above defined regulatory nucleotide sequence, which is functionally linked to a determined nucleic acid sequence of interest.

By the expression "functionally linked", it is meant that when the regulatory nucleotide sequence is used in combination, for instance in a fusion, with the nucleic acid sequence of interest, and when possible additional elements involved in the regulatory activity are present or activated, a regulatory activity enabling the transcription of said nucleic acid interest is obtainable in competent host cells.

According to a particular embodiment, the invention relates to a recombinant nucleotide sequence, wherein the regulatory nucleotide sequence is functionally linked to the nucleic acid of interest and wherein this construct further comprises 3' from the nucleic acid of interest, a non translated sequence comprising a transcription termination sequence. This transcription termination sequence can especially contain polyadenylation sites.

The nucleic acid sequence of interest can be of various type or origin especially originating from plants, animals, bacterial, viral organisms. It is especially the sequence encoding the I-2 resistance gene product.

Alternatively, said nucleic acid sequence of interest can be chosen among sequences having a direct or indirect activity in resistance against pathogens, especially resistance against plant pathogens, particularly those having a targeted tissue specificity. As example, resistance can be mediated through an interaction with the site of penetration of the pathogen, or of the infection capacity of the pathogen or through an effect on the colonization of tissues in infected plants.

In accordance with the invention, the nucleic acid sequence of interest can be chosen among sequences which are naturally expressed in a tissue specific manner in their natural host, or for which no tissue specific expression exists in the natural host. In this latter case especially, a tissue specific expression would be specifically obtained by the use of the regulatory sequence of the invention when it is combined to the chosen sequence, said chimeric construct being then transferred into a host plant or plant host cell.

The invention is especially directed to the use of the recombinant nucleotide sequence defined above for a specific expression in a plant vascular tissue. According to the invention, the expression "plant vascular tissue" encompasses tissues having vascular cells whatever their location in the plant. Vascular tissues are especially present in roots, stems or leaves. The expression therefore encompasses cells, or cell layers from vascular origin or having a vascular related function. It comprises cell layer(s) surrounding the lateral root primordia or cell layers at the base of adventitious roots, or cells in the leaf veins, in the plant fruits or vegetable, or in the stigma of the style.

The inventors have shown that the use of the regulatory sequences of the invention may be of specific interest for the expression of nucleic acid sequences in plant lateral root meristem and tissues deriving from said meristem.

Among sequences which are regarded as sequences of interest to be expressed in a tissue specific manner within host plants, genes or generally DNA constructs having activity in mediation of resistance to a plant pathogen are preferred.

Among these plant pathogens, those qualifying as root pathogens and accordingly invading plants through the roots, are of interest. As an example, fungi, nematodes are of interest and especially fungi of Fusarium species are cited.

The recombinant nucleotide sequences of the invention can be used for transfer in plants or in plant cells, according to any available technique. Particularly recourse may be made to the use of vectors especially plasmids or cosmids, containing said recombinant nucleotide sequence for transforming plant cells or plants.

The invention therefore is directed to recombinant cells comprising a nucleotide sequence defined above, including recombinant nucleotide sequences according to the invention, or vectors containing the same.

Among appropriate plant cells which can be chosen as targets for the expression of the nucleic acid of interest placed under the control of the regulatory sequences of the invention, bacterial or eukaryotic cells can be cited.

Among eukaryotic cells, most preferred cells are plant cells and especially plant cells originating from vascular tissues.

According to another embodiment of the invention, said plant cells are root cells, stem cells or leaf cells.

The invention also relates to plants which are recombined with the nucleotide sequences which have been disclosed above.

According to a specific embodiment of the invention, said recombinant plants are capable of expressing a gene having a mediation activity of resistance to a plant pathogen. Particularly, plant pathogens among those cited above are preferred and especially when they are known to have a tissue specific invading activity.

Recombinant plants according to the invention can be derived from recombination with the nucleotide sequences of the invention of tomato, egg plants, potato, melon, tobacco or Arabidopsis.

The invention is also directed to a process for obtaining plants having reduced susceptibility to a plant pathogen, comprising the following steps:
i) inserting into the genome of a plant cell a nucleotide sequence defined above, or a vector defined above,
ii) obtaining transformed plant cells,
iii) regenerating from said transformed plant cells genetically transformed plants, and
iv) optionally, propagating said plants.

The above defined process is of specific interest to obtain transgenic plants which are further selected for having a reduced susceptibility to a plant pathogen as a result of the expression of the nucleic acid of interest under the control of the regulatory sequences of the invention.

The invention is also directed to a process for protecting plants in cultivation against plant pathogen infection, which comprises:
i) providing the genome of plants with a nucleotide sequence defined above, or a vector defined above,
ii) growing said plants.

Further details and advantages regarding the invention appear from the following examples and figures.

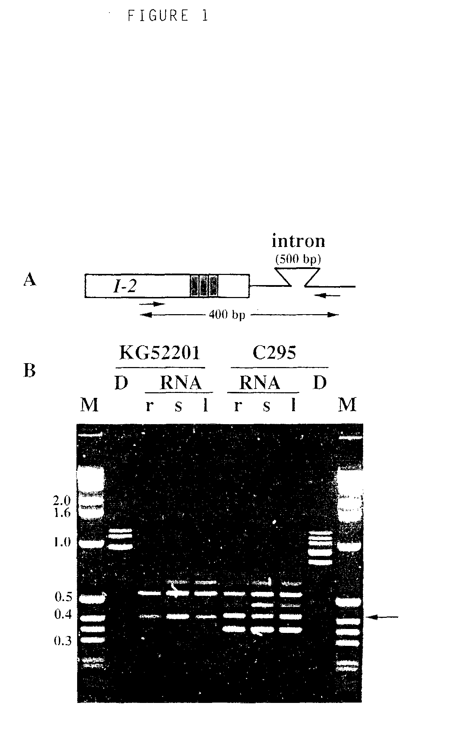

**Fig. 1.** Transcripts of *I-2* and *I-2* homologs detected by RT-PCR. **A**, Schematical representation of the *I-2* region used for cDNA amplification. Primers are indicated by arrows, gray boxes are the region of variable repeats between *I-2* and *I-2* homologs. **B,** The by RT-PCR amplified fragments. KG52201 is Fusarium susceptible line; C295 is *I*-2 containing Fusarium resistant line; M=marker (kb); D=DNA; r=RNA from root; s=RNA from stem; I=RNA from leaf; arrow indicates expected lenght *I-2* transcript.

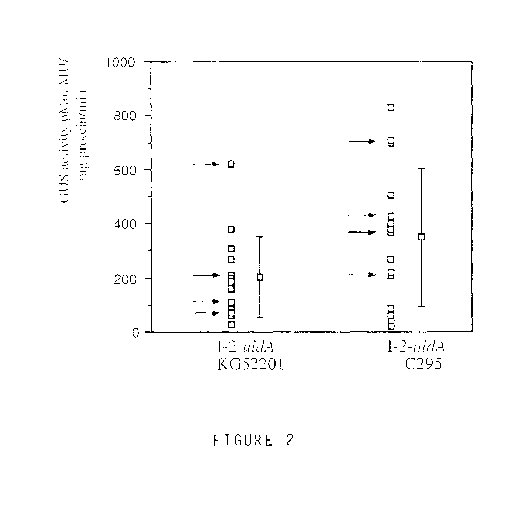

**Fig. 2.** Comparison of GUS activity in seedlings of transgenic tomato plants of line KG52201 and C295. Each of the 15 squares per line represents the activity of 5 pooled nonselected seedlings of 15 individual transgenic lines. The mean and standard deviation of the 15 samples are presented besides. Arrows points out lines used for further analysis.

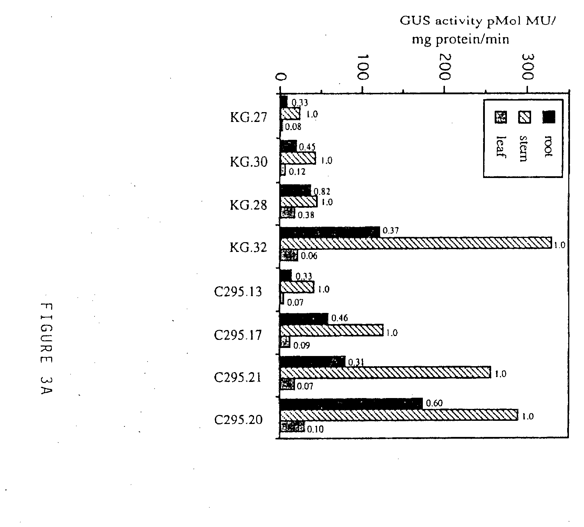

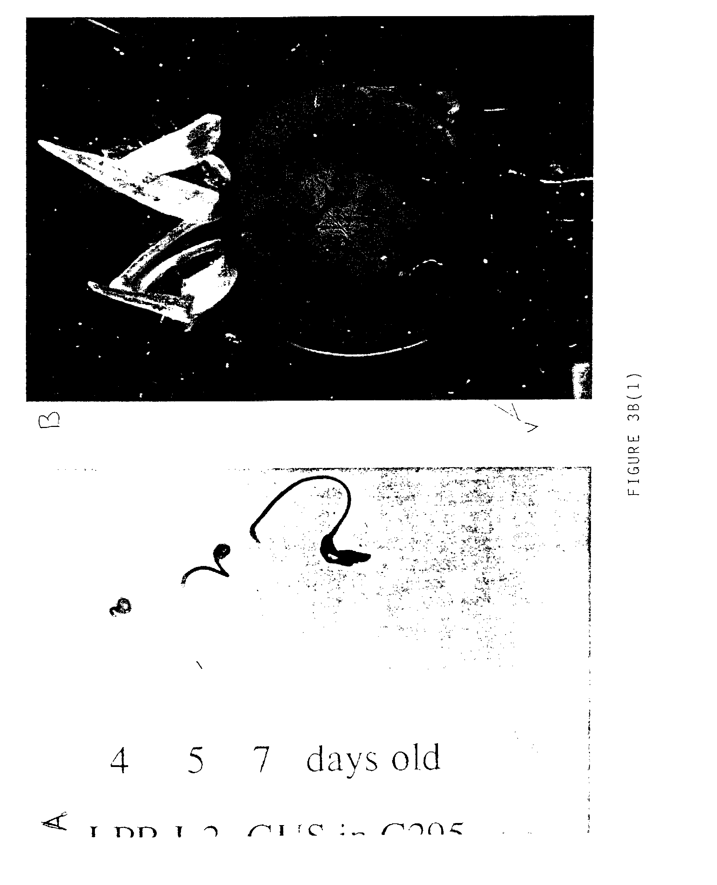

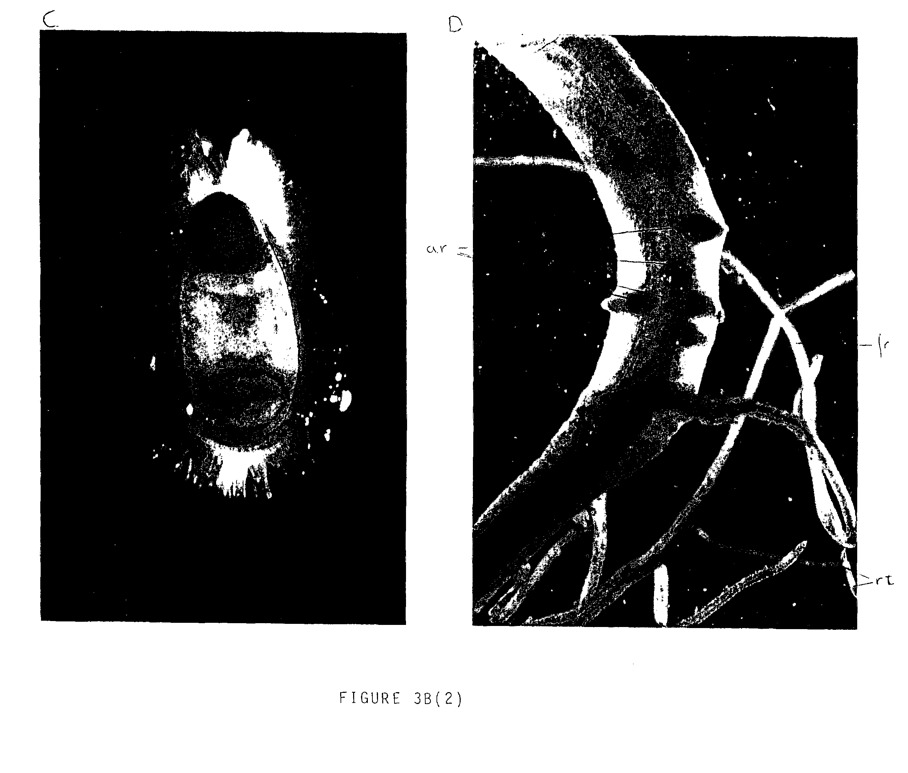

**Fig. 3A.** Comparison of GUS activity in roots, stems and leaves of transgenic lines. Bars represent the expression of material pooled from 10 selected transgenic plants.

**Fig. 3B.**
**A. seedlings 4, 5 and 7 days-old**
**B. tomato fruit (not ripe)**
   V= vascular bundle
**C. seed**
   H=hypocotyle, C=cotyledons
**D. stem**
   AR=adventitious root (premordia), Fr=fibrious root, Rt=root tip
**E. vascular bundle of stem**
   Co=cortex, E=endodermis, Pi=pericycle, Ca=cambium, Ph=phleom, Xp=xylem parenchyma, X=xylem mature
**F. central cylinder of root**
   Co=cortex, E=endodermis, Ca=cambium, Ph=phleom, Xp=xylem parenchyma, X=xylem mature, Px=premature xylem
**G. lateral root formation**
   Lr=lateral root, V=vascular tissue, Rh=root hairs
**H.leaf, I-2 expression upper leaf, under 35SCaMV leaf**
   Ve=veins

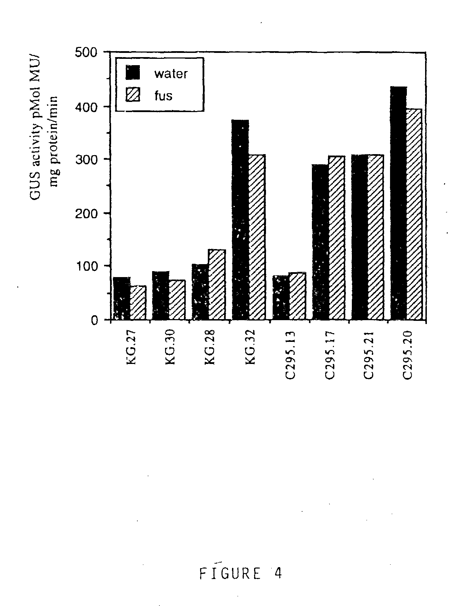

**Fig. 4.** Comparison of GUS activity in stem of Fusarium infected and water treated transgenic plants. Bars represent the expression of 20 pooled stems of nonselected transgenic plants.

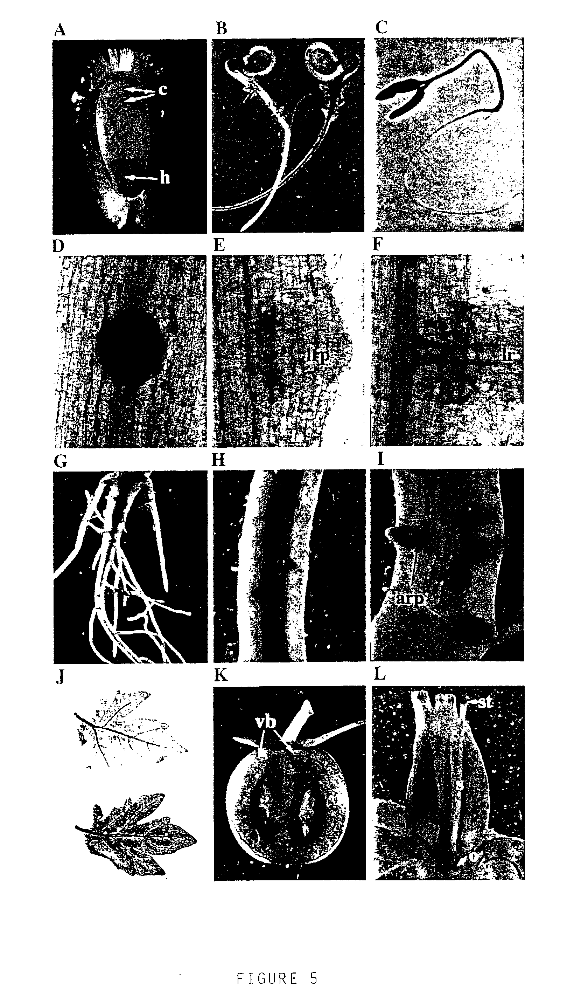

**Fig. 5.** Macroscopical localization of *uidA* gene expression under control of the 5' flanking region of the *I-2* gene. **A**, Seed. **B**, Germinated seed, right I-2 promoter, left 35SCaMV promoter. **C**, 7-days-old seedling. **D**, Lateral root primordia, top view. **E**, Lateral root primordia, side view. **F**, Base of lateral root, side view. **G**, Mature tap root. **H**, Stem.**I**, Adventitious root formation. **J**, Leaf, upper I-2 promoter, bottum 35SCaMV promoter. **K**, Tomato fruit. **L**, Flower. c = cotyledons, h = hypocotyl, Irp = lateral root primordia, r = root, arp = adventitious root primordia, vb = vascular bundle, st = stigma, s = style, o = ovary.

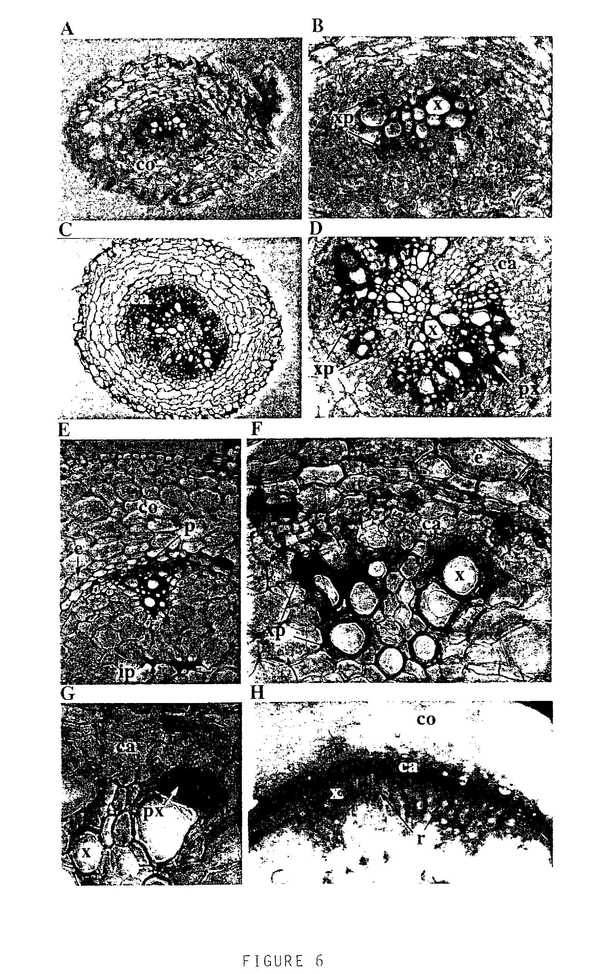

**Fig. 6.** Histochemical localization of *uidA* gene expression under control of the 5' flanking region of the *I-2* gene. **A**, Young root. **B**, Vascular tissue of young root. **C**, Mature tap root. **D**, Vascular tissue of tap root. **E**, Stem section. **F**, Detail of stem. **G**, Detail of vascular bundle of stem. **H**, Stem section of three-weeks-old plant. vt = vascular tissue, ca = cambial zone, co = cortex, xp = xylem parenchyma, x = xylem, px = premature xylem, e = endodermis,
p = phloem, ip = inner phloem, r = rays.

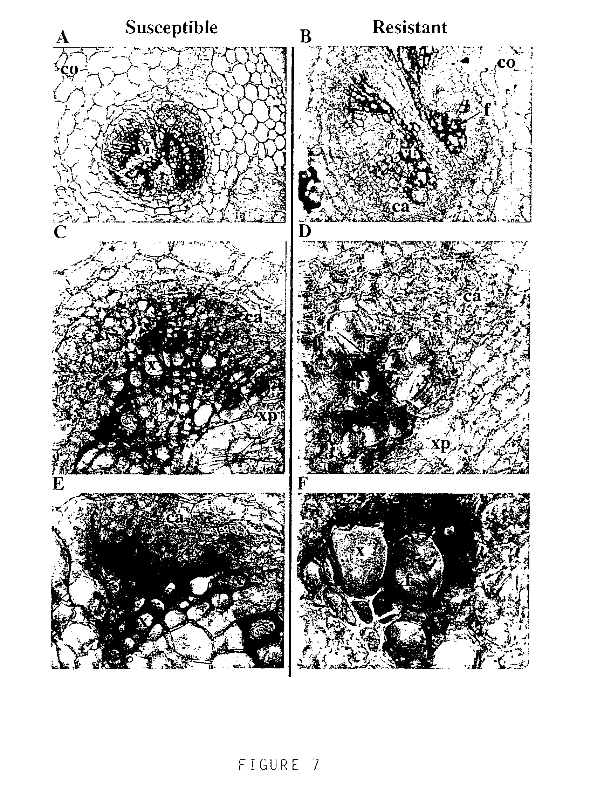

**Fig. 7.** Histochemical localization of *F. oxysporum* f.sp. *lycopersici* infection by using constitutive expressing *uidA* transgenic *Fusarium* isolate, seven days after inoculation. **A**, Infected root of susceptible plant. **B**, Infected root of resistant plant. **C**, Detail of infected root of susceptible plant. **D**, Detail of infected root of resistant plant. **E**, Stem section of susceptible plant. **F**, Stem section of resistant plant. co = cortex, vt = vascular tissue, f = *Fusarium,* ca = cambial zone, x = xylem, xp = xylem parenchyma.

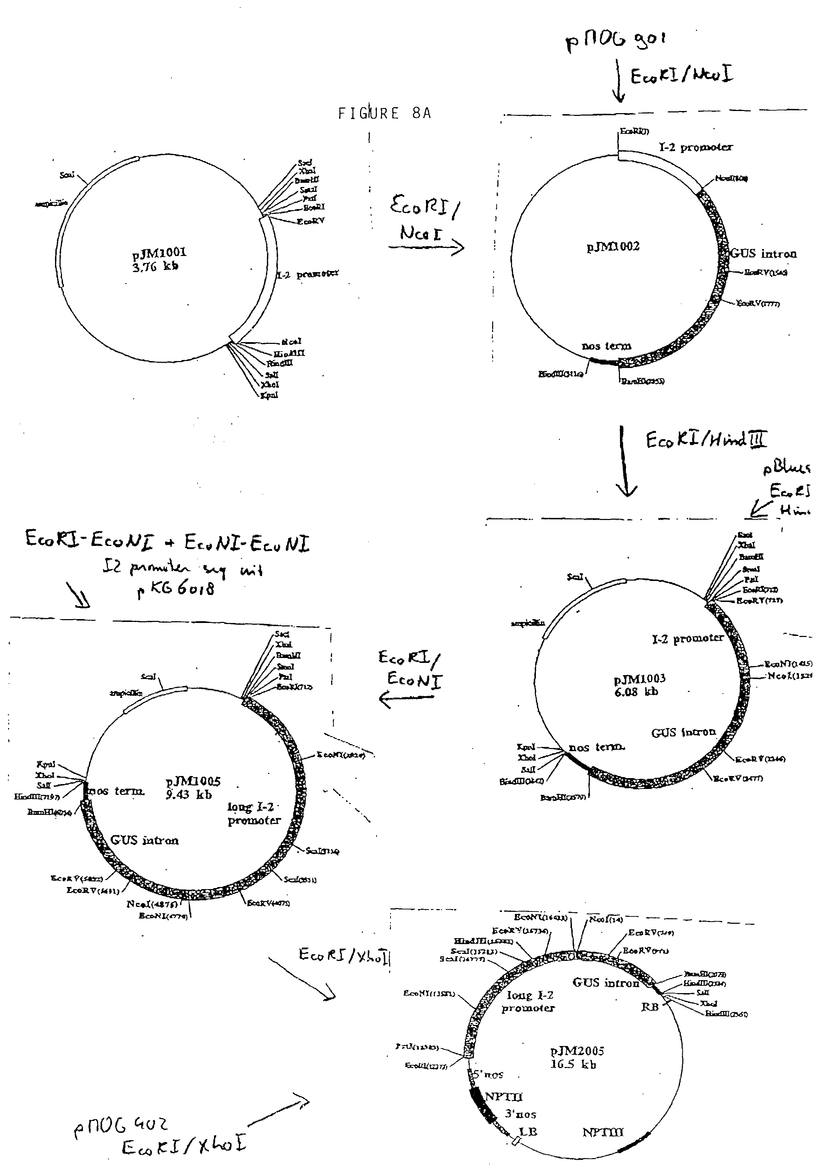

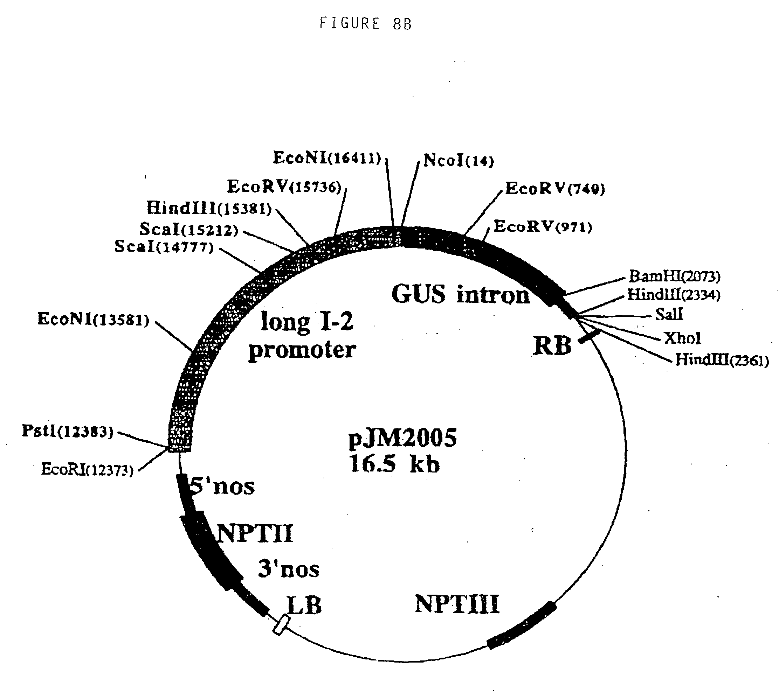

**Fig. 8.** Constructs
A: Steps for the construction of pJM2005
B: pJM2005 containing an I-2 promoter *uid* A chimeric gene with long I-2 promoter sequence (3.8 kb)

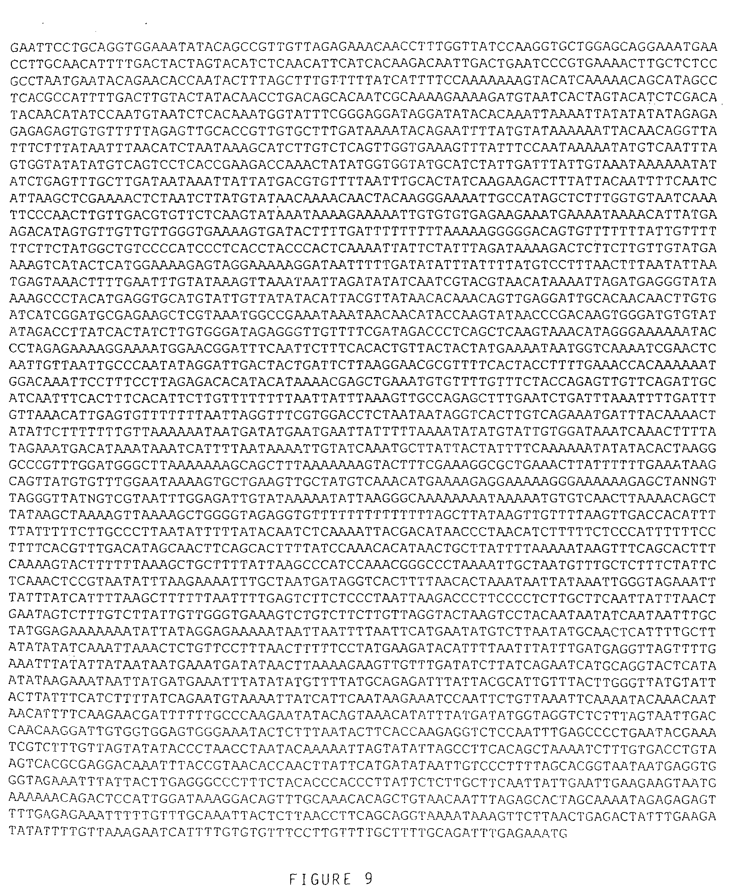

**Fig. 9.** Sequence of the 3.8 kb DNA non coding fragment.

### Expression of the Fusarium I-2 resistance gene co-localizes with the site of defence response

The expression of the *Fusarium* resistance gene *I-2* of tomato has been analysed. Although *I-2* transcripts were undetectable by Northern blot analysis, RT-PCR revealed that *I-2* and at least five *I-2* homologs are expressed in roots, stems and leaves of young tomato plants. Plants transformed with chimeric constructs containing a functional *I-2* promoter fused to the β-glucuronidase (GUS) reporter gene were used in detailed expression studies. GUS-activity was found in unchallenged plants; infection with *F. oxysporum* f.sp. *lycopersici* race 2 did not alter promoter activity. Macroscopical analysis showed that the *I-2* promoter drives expression of the reporter gene in a cell layer beneath the lateral root primordia, in mature roots, at the base of adventitious roots and in vascular tissue of stems, leaves and fruits. The vascular specific expression was confirmed by microscopical analysis, which revealed expression in endodermis, cambial zone, phloem, xylem parenchyma cells, premature xylem vessels and rays cells. We show that in resistant plants fungal growth into this region of the vascular tissue is prevented suggesting a direct role of *I-2* in mediation the resistance response of tomato to *F. oxysporum* f.sp. *lycopersici.*

Tomato may become infected by the soil-borne pathogen *Fusarium oxysporum* f.sp. *lycopersici.* The fungus enters the plant via the roots either by active penetration at the root tip (Bishop and Cooper, 1983) or via natural wounds, for instance via cortex tissue that is disrupted by the formation of lateral roots (Hutson and Smith, 1983). Penetration of vascular elements depends on the presence of cells that are injured, senescent or necrotic, or circumstances providing entry through wounds (Beckman, 1987). In xylem vessels the fungus spread by long distance transport using the transpiration stream (xylem flow). Fungal spores germinate within the vascular elements, grow towards pits and colonize adjacent xylem parenchyma cells. Colonization of secondary xylem parenchyma cells has been shown to be typical for susceptible plants (Beckman et al., 1989). These living xylem-contact-cells are held responsible for defence responses in lateral and longitudinal directions, including callose deposition, production of secondary metabolites, lignification and vascular occlusion by gels, gums or tyloses (Beckman, 1987; Beckman and Roberts, 1995). Defence responses of susceptible and resistant plants are basically the same, only the timing and strength of the response is different (Beckman, 1987).

The dominant resistance gene *I-2* of tomato confers resistance to F. *oxysporum* f.sp. *lycopersici* race 2. An AFLP-based positional cloning strategy was used to clone the *I-2* gene which is located within a cluster of seven homologs (Simons et al., 1998). The *I-2* gene encodes a protein with similarities to a large family of *R* gene products containing a nucleotide binding site and a leucine-rich repeat (NBS-LRR) (Simons et al., 1998; Hammond-Kosack and Jones, 1997; Ellis and Jones, 1998). The structural domains predict a role in signalling, transducing a signal from the invading pathogen to the activation of defence related genes (Hammond-Kosack and Jones, 1997; Ellis and Jones, 1998).

The characterization of *I-2* promoter activity could help to localize the actual site of the resistance reaction. How resistance genes function and whether these genes encode other functions in plants apart from mediation of resistance, is not known. Gene expression analysis could lead to a better understanding of the resistance mechanism as well as to suggestions for other functions of *R* genes. For none of the cloned resistance genes such characterisations have been published until now. To analyse *I-2* promoter activity, tomato plants were transformed with chimeric constructs containing a functional *I-2* promoter region fused to the β-glucuronidase (GUS) reporter gene. Macroscopical and histochemical analysis revealed a specific expression in cells neighbouring the xylem vessels, suggesting a very specifically localized defence reaction against *F. oxysporum* f.sp. *lycopersici.*

### MATERIAL AND METHODS

**Bacterial and fungal strains**. *E.coli* strain DH5a was used for plasmid propagation. Infection test were performed using *F. oxysporum* f.sp. *lycopersici* race 2 isolate Fol007.

**Plant material.** Tomato line KG52201 was used as susceptible line containing no resistance to any race of *F. oxysporum* f.sp. *lycopersici.* Line OT105 (E22) was used as a control line because the *I-2* resistance gene was cloned form this line (Simons et al., 1998). OT105 is homozygous, and is resistant to *F. oxysporum* f.sp. *lycopersici* race 2 and susceptible to race 1. Moneymaker line C295 is resistant to *F. oxysporum* f.sp. *lycopersici* race 1 and race 2.

For RNA isolation plant tissue was grounded in liquid nitrogen. After addition of extraction buffer (100 mM Tris-HCI pH 8.5, 100 mM NaCI, 20 mM EDTA and 1% sarkosyl) RNA was extracted with phenol and twice with phenol/chloroform (1:1) before it was precipitated witih 2-propanol. DNA/RNA pellet was dissolved in water and RNA was precipitated by addition of an equal volume of 4M LiCI. The RNA pellet was dissolved in H₂O, precipitated with ethanol and redissolved in 20-100µl H₂O. Ten µg of total RNA was separated on 1.5% denaturating formaldehyde agarose gel electrophoresis and subsequently blotted on Hybond-N membrane (Amersham). The I-2 probe was ³²P-labelled by the Random Primers Labelling System (BRL Life technologies Inc., USA) and hybridization was performed in 0.5M phosphate buffer pH 7.2 containing 7% SDS, 1% BSA and 1mM EDTA. After overnight incubation at 65°C, the blot was washed with 2x and 1x SSC, respectively, for 15 min each.

For cDNA synthesis 50 ng oligo dT primer was added to 5 µg of RNA in a volume of 6.2 µl. After annealing, 3.8 µl was added containing 0.4 µl dNTP 10 mM, 1 µl DTT 100 mM, 2 µl 5x RT buffer, 0.2 µl RNAse inhibitor (30U/µl) and 0.2 µl Superscript II (200U/µl) (Gibco-BRL). The mixture was incubated at 45°C for one hour. PCR was performed under standard conditions using 2 µl of cDNA. Primers 3449 (5' CCTCCTTTTCTCACCTCACTCGC 3') and PF95 (5' GTACCAGACTTGTCGTACTCGCTC 3') were used to amplify the 3' end of both the active *I-2* gene and other *I-2* homologs.

**Constructs.** To construct an *I-2* promoter *uidA* chimeric gene, an *Ncol* restriction site was created at the translation start of *I-2*. This resulted in a change of two bases upstream of the translation initiation codon from AA to CC. The *Ncol* site was introduced by PCR using primer FP4 (5' CTGCTAAGCTTATCT**CCATGG**CTCAAATC 3') together with primer FP3 (5' GTTGTTTGATATCTTATCAG 3') spanning an EcoRV restriction site located 806 bp upstream of the ATG. The fragment was cloned in pBluescript which then was called pJM1001 (see figure) and sequencing confirmed that it was identical to the original sequence of the 5' flanking region of *I-2*. An *Eco*RI*/N*col fragment, containing the 806 bp PCR I-2 promoter sequence of pJM1001 was used to exchange the *Eco*RI*/N*col 35S promoter fragment of pMOG901 (ZENECA-MOGEN, Leiden, the Netherlands). This resulted in plasmid pJM1002 containing the 806bp I-2 promoter fragment fused to the β-glucuronidase gene (*uidA* with modified intron) with the *nos* terminator region. To introduce more cloning sites the 805bp 1-2 promoter-GUS fragment was *Eco*RI/*Hin*dIII cloned in pBluescript KS+ (pJM1003). An *Eco*RI/*Eco*NI fragment of 3.7 kb of the 5' flanking region of *I-2* was used, in a two step cloning, to partial exchange the PCR based 806 bp sequence (105 bp PCR fragment remained but which was identical to the original sequence) and extend the promoter sequence. This resulted in pJM1005 containing 3.8 kb 5' flanking region of the *I-2* gene fused to *uidA* gene and the *nos* terminator. This construction enables to make a restriction site at the ATG codon therefore enabling to make constructs which start directly at the ATG codon.

Using EcoRI and Xhol the 3.8 kb I-2 promoter-GUS fragment was cloned into the binary vector pMOG402 (ZENECA-MOGEN, Leiden, The Netherlands) containing the Left and right border, PPTII regulated by nos 5' and 3' sequences. This plasmid was called pJM2005.

Using the same strategy, an *Nco*l site was introduced at the ATG of the *I-2* coding region using primers FP70 (5' CAGATTTGAG**CCATGG**AGATTG 3') and FP71 (5' GCTGACCTTCCACCTTAAG 3'). The first primer introduces the *Nco*l site, the second is spanning a *Sal*I restriction site for further cloning strategies. The amplified fragment was cloned, sequenced and used to exchange the *I-2* sequence in pKG 6016.

In a three point ligation the *Eco*RI*/N*coI promoter fragment of pJM1005 and the *Ncol/Xhol* 3801 bp coding region of *I-2* and 1100 bp *I-2* downstream sequence were cloned into the *EcoRI/Xho*l digested pMOG402, resulting in pMH4002.

**Plant transformation**. The binary T-DNA vectors pJM2005 and pMH4002 were introduced into *Agrobacterium tumefaciens* strain EHA105 by electroporation. Constructs were checked for recombination events by transformation of *E.coli* DH5α with plasmid isolated from transformed A. *tumefaciens* followed by restriction analysis. Tomato cotyledons were transformed essentially as described by Fillatti et al. (1987). Ploidy level of transformants was checked by counting the number of chloroplasts in the stomatal guard cells.

**Infection assay**. Infection assays were performed as described previously (WO 97/06259).

**Quantitative GUS assay.** GUS activity was quantified fluorometrically by using the substrate 4-Methylumbelliferone glucuronide (MUG) as described by Jefferson (1987). Reactions were performed in 50 µl, containing 1 mM MUG and stopped after 2 hours incubation at 37°C by addition of 0.5 M Na₂CO₃. The fluorescence was related to a calibration curve of 4-methylumbelliferone (MU). Protein determination was carried out using Bradford reagents and BSA as standard.

**Histological observation.** GUS assays were performed histochemically with 5-bromo-4-chloro-3-indoyl glucuronide (X-Gluc) according to Jefferson (1987) and modified by Toriyama et al. (1991). Plant tissues were emerged in the GUS staining solution (1 - 0.5 mM X-Gluc, 0.1 M sodium phosphate buffer, pH 7.0, 0.5% Triton X-100, 2 mM ferrocyanide and 2 mM ferrycyanide) and vacuum infiltrated for at least one hour in total. Incubations were at 37°C overnight after which plant material was fixed in FPA (Formalin 2%, Propiono acid 5% in ethyl-Alcohol 63%) and subsequently incubated in 70 % ethanol. Root and stem sections were embedded in paraffin and sectioned at 120 µm using a sledge microtoom.

### RESULTS

### Expression of the I-2 gene family

Northern blot analysis on total RNA from roots, stems and leaves of *I-2* containing tomato (line C295) did not give any hybridization signal using *I-2* DNA as probe. RNA isolated from Fusarium-infected plants did not yield a signal either. These results suggest that the transcription level of *I-2* and other members of the *I-2* family is very low. Alternatively, *I-2* transcripts may be very unstable. In a next attempt to detect *I-2* transcripts an RT-PCR approach was followed. Homologs of *I-2* differ from the active gene by the number of a completely conserved 23 amino acid repeat (Simons et al., 1998). Within *I-2* three tandemly arranged copies of this repeat are present, whereas in homologs the number of copies vary from two to six. A set of two primers was designed spanning a sequence containing the region with this variable number of repeats and the intron in the 3' UTR (Fig. 1A). Using this primer set fragments amplified on genomic DNA can be distinguished from fragments amplified on cDNA, and the *I-2* transcript can be distinguished from transcripts of *I-2* homologs (Fig. 1A). On genomic DNA as template the presence of at least three homologs could be demonstrated in susceptible line KG52201 and at least five homologs in the resistant line C295 (Fig. 1 B, lanes D). As expected, fragments amplified on cDNA are smaller due to the absence of the intron. RNA isolated from roots (r), stems (s) or leaves (I) of young plants yielded at least six transcripts in the *I-2* containing line C295 and three *I-2*-like transcripts in susceptible line KG52201. The expected size of the *I-2* fragment is indicated by an arrow. This result shows the presence of *I-2* transcripts in root, stem and leaf in the absence of a *Fusarium* infection. The low abundancy of transcripts and the expression of various homologs with indistinguishable 5' and 3' ends prompted us to use *I*-2 promoter-GUS fusions to study the expression of *I*-2.

### Functional expression of I-2 in transgenic plants

To investigate the functionality of the *I-2* promoter fragment to be used in expression studies, a construct was made consisting of a 3.8 kb 5' flanking sequence, the *I-2* coding region and a 1.1 kb 3' flanking sequence. This construct was used to transform the race 1 and 2 susceptible tomato line KG52201. Seventeen individual transformants were selected and selfed. R1 lines were tested for resistance to *F. oxysporum* f.sp. *lycopersici* race 2 in standard root dip inoculation experiments. To this end 20 plants of each line segregating for the transgene were tested. Plants were inoculated and potted separately to enable a reliable quantification of resistance by measuring the weight of plants three weeks after infection (WO 97/06259). Table 1 shows the results of a representative experiment. Control lines show susceptibility (KG52201) or resistance (OT105 and C295) as expected. Two (KG.14 and KG.05) out of seven transgenic lines tested were found to be resistant to *F. oxysporum* f.sp. *lycopersici* race 2: after infection their mean weights are significantly (p=0.001) different from the susceptible control and are almost as high as the water control. KG.14 and KG.05 showed the expected 3:1 (resistant : susceptible) segregation of the resistance trait. The other five lines containing the same construct either did not differ significantly from the susceptible control line (KG.15) or showed such a reduction in plant weight compared to the water control that they all must be considered susceptible (Table 1). In total five lines (29%) of the 17 lines tested were found to be resistant. From this we conclude that for functional expression of the *I-2* resistance gene the 3.8 kb promoter fragment is sufficient.

**Table 1.**

| Resistance of transgenic lines complemented with the *I-2* gene flanked by 3.8 kb 5' flanking region and 1.1 kb 3' downstream region. | | | | |
|---|---|---|---|---|
| Tomato line | Water treated weight (g) | Fusarium infected weight (g) | | Segregation (R:S) |
| KG52201 | 15.8±1.5 | 0.8±1.6 | a | 0 : 20 |
| KG.15 | 12.5±2.1 | 2.4±2.9 | ab | 0 : 18 |
| KG.13 | 12.0±1.2 | 2.6±3.0 | b | 1 : 19 |
| KG.16 | 12.7±1.4 | 3.4±2.8 | b | 0 : 20 |
| KG.18 | 13.2±1.6 | 3.5±3.3 | b | 2:18 |
| KG.02 | 11.7±1.6 | 3.7±3.1 | b | 1:19 |
| | ± | ± | | |
| KG.14 | 12.7±2.6 | 9.1±3.7 | c | 14 : 5 |
| KG.05 | 12.0±0.5 | 10.0±4.1 | cd | 15 : 3 |
| OT105 | 17.1±1.5 | 11.8±1.5 | d | 20 : 0 |
| C295 | 18.0±1.6 | 14.3±1.1 | e | 19 : 0 |

### I-2 promoter driven expression of the GUS reporter gene

Transgenic lines containing the 3.8 kb 5' flanking sequence of the *I-2* gene fused to the *uidA* (GUS) reporter gene were analysed for expression. R1 seedlings of 15 individual transgenic lines of both KG52201 and C295 were grown in potting soil. Five plants per line were pooled and tested for expression using the quantitative MUG assay. Between transgenic lines expression levels appeared to be highly variable (Fig. 2). Mean expression levels were higher in C295 transgenic than in KG52201 lines, although the differences were not found to be significant (p=0.065). Of each cultivar four transgenic lines showing different levels of expression were selected for further analysis. Roots, stems en leaves of kanamycine resistant seedlings were pooled and tested separately. Of each transgenic line material of ten plants was pooled. The variation in GUS activity (Fig. 3) between the different transgenic lines correlated with the results obtained before (Fig. 2). In all lines GUS activity was found to be highest in stems and lowest in leaves.

### Histochemical analysis of tissue specific expression of the I-2 promoter in unchallenged plants

Eight lines transgenic for I-2-gus-nos of both cultivars KG52201 and C295 were selected for detailed histochemical analysis. These transgenic plants all expressed the transgene in an identical patter, although expression levels seem to vary. In most cases a lower intensity of blue staining was found in the transgenic lines of K52201.

Seeds and seedlings were analysed for GUS activity. The seed embryo showed GUS expression in hypocotyl and cotyledons. The same was found when the seeds had germinated into young seedlings, but no expression was detectable in the roots on microscopical level. In a later stage, young roots showed a very faint staining in some of the scarce xylem parenchyma cells. In these young roots, a very pronounced expression was found at the origin and base of lateral root formation. In an early state of lateral root primordia, GUS staining was visible as a circle. When lateral root primordia futher developed in a lateral root, expression remained visible at the root base where it has escaped from the older root. The signal becomes more diffuse during growth of the root, probably by dilution of the initial expression because of cell elongation. When secondary thickening of the root has occurred resulting from cambial activity, high GUS activity could be detected. Low activity was found in the endodermis, phloem and vascular cambium. A very pronounced activity could be observed in xylem parenchyma cells surrounding the xylem vessels as well as in premature xylem vessels where cytoplasm was still present. In primary meristem of the root tips no expression was found.

Expression in stem was detected both in young and old plants. The GUS activity was localized in the same tissue as described for the root: endodermis, phloem and cambial tissue xylem parenchyma cells and xylem vessels when still containing cytoplasm.

Secondary lateral growth of the stem resulted in more cambial tissue and xylem vessels, both showing pronounce GUS staining. Especially, the ray parenchyma cells, the living cells that are located adjacent to the secondary xylem show a pronounced GUS activity. Analogue to the expression of GUS by the I-2 promoter at the base of lateral root formation, expression was abundantly present at the base of adventitious roots which originate from the stem.

The I-2 promoter also drives GUS expression at the leaf veins, likely with the same tissue specificity as described for root and stem. That the staining is not due to uncompleted substrate infiltration is shown by the comparison with a 35SCaMV transgenic leaf which revealed GUS activity in the complete leaf. The vascular specific expression of I-2 is also found in the tomato fruit. Staining can be observed in the whole fruit but is most abundantly present in the vascular veins. Surprisingly, the I-2 promoter is also active in the stigma of the style.

| | **I-2 promoter** | **35SCaMV** |
|---|---|---|
| seed hypocotyl | + | + |
| | | |
| young (fibrious) root | - | + |
| tap root and secondary thickened roots | + | + |
| adventive root primordia base | + | - |
| adventive root (fibrious root) | - | + |
| adventitious root base | + | - |
| | | |
| young stem | + | + |
| older stem | + | + |
| exodermis | - | + |
| cortex | - | + |
| endodermis | + | + |
| pericyle | - | + |
| cambium | + | + |
| phloem (internal and secondary) | + | + |
| xylem parenchyma | + | + |
| xylem (premature) | + | ? |
| ray parenchyma cells (pith rays) | + | + |
| hairs | - | + |
| | | |
| leaf mesophyll | - | + |
| leaf vein | + | + |
| gladular trichomes | + | - |
| stigma of pistil | + | + |
| tomato fruit | + | + |

To examine induction of expression by *Fusarium,* the same eight lines were used. Of each line 20 R1 seedlings that had not been selected for the presence of the transgene, were infected with *F. oxysporum* f.sp. *lycopersici* race 2 by a standard root dip method or were mock inoculated with water. Seven days after infection, when susceptible plants started to show Fusarium wilt symptoms, stems of the seedlings were analysed for GUS activity. No induction nor suppression of GUS activity was found in inoculated plants (Fig. 4). From these results we conclude that *I-2* promoter driven expression is not changed by *Fusarium* infection. However, we cannot yet exclude changes in expression levels in tissue around the site of colonization since such changes can not be detected with this type of experiments.

### Macroscopical analysis of GUS expression

The transgenic lines used before of both cultivar KG52201 and C295 were used for analysis of the tissue specificity of expression. In all cases expression patterns were identical, although levels varied. Since C295 lines showed most pronounced GUS staining, these lines were used in further studies. The seed embryo displayed GUS activity in hypocotyl and cotyledons (Fig. 5A). The same was found when seeds had germinated (Fig. 5B and C). At macroscopical level, no expression was visible in the roots. For comparison CaMV 35S promoter driven GUS expression in roots is shown (Fig. 5B, right-hand seedling). At sites where tap roots developed lateral roots, a very pronounced expression was found in a cell layer beneath the lateral root primordia (Fig. 5D; E and F). In an early stage of lateral root formation, GUS staining was visible as a circle when viewed from above (Fig. 5D). A side view showed the expression in a single layer of cells beneath the lateral root primordia (Fig. 5E). When these primordia further developed into lateral roots, expression remained visible, in particular at the root base where it had emerged from the older root (Fig. 5F). The signal became more diffuse during growth of the root probably because of cell elongation. GUS activity could be detected in mature roots that had grown by secondary thickening (Fig. 5G). In the primary meristem of root tips no expression was found. Expression in the hypocotyl was visible both in young and old plants. GUS activity was localized in the central part of the stem, most likely in the vascular tissue (Fig. 5H). In analogy with expression of the GUS gene at the base of lateral root primordia, expression was also found at the base of adventitious roots originating from the stem (Fig. 5I). The *I-2* promoter drives GUS expression in leaf veins as well (Fig. 5J, upper leaf), again demonstrating vascular specificity. For comparison 35S promoter driven GUS expression in the leaf is shown as well (Fig. 5J, bottom leaf). Vascular specific expression is also found in tomato fruit where staining is most abundantly in the vascular bundles (Fig. 5K). Finally, the *I-2* promoter is active in the stigma of the style (Fig. 5L).

### Histochemical analysis of expression of the GUS reporter gene

In young roots low GUS activity was found in the few xylem parenchyma cells and premature xylem vessels (Fig. 6A and B). In older roots in which xylem tissue has expanded because of cambial activity, primarily vascular tissue was stained (Fig. 6C and D). Detailed analysis revealed expression in the cambial zone, xylem parenchyma cells and premature xylem vessels where cytoplasm was still present (Fig. 6D). In stems, where the central xylem developed into separate vascular bundles, GUS activity was localized not only in the same tissues as described for the root: cambium, xylem parenchyma cells and premature xylem vessels (Fig. 6E and F). but also in the endodermis, the phloem and inner phloem vessels. Remarkably GUS activity is highest in premature xylem vessels in all tissues analyzed (Fig. 6G). Secondary lateral growth of the stem results in more cambial tissue, xylem parenchyma cells and xylem vessels (Fig. 6H). Especially the cambial zone and the rays, the living cells that are located adjacent to the secondary xylem, show a pronounced GUS activity.

### Histochemical localisation of Fusarium in infected tomato.

Detailed studies of *F. oxysporum* f.sp. *lycopersici* colonizing tomato have been published (Beckman 1987; Beckman and Roberts 1995). However, to be able to correlate the *I-2* promoter activity in the same experimental design as presented in the previous section, we analyzed the colonization of tomato by a GUS marked Fusarium. Non-transgenic KG52201 (susceptible) and C295 (resistant) plants were inoculated with a transgenic *F. oxysporum* f.sp. *ycopersici* race 2 isolate expressing the GUS gene driven by the constitutive glyceraldehyde-3-phosphate dehydrogenase promoter (Roberts et al., 1989). Three, five and seven days after inoculation, plants were harvested for detailed histochemical analysis. Roots of both susceptible and resistant plants were colonized by F. *oxysporum* f.sp. *lycopersici* within three days. Five days after inoculation, roots and hypocotyl of all susceptible plants were colonized and colonization went on until day seven. Three days after inoculation the colonization was also visible in xylem vessels and expanded to xylem parenchyma cells of the roots, and often even further into the cambial zone at days five and seven (Fig. 7A and C). In resistant plants roots showed a much lower level of GUS activity and in only a few cases (10% of the plants observed) localized GUS staining was found higher up in the tap root or in the stem. Microscopical analysis showed that the fungus was restricted to the xylem vessels and occasionally to a single layer of xylem parenchyma cells (Fig. 7B and D). Around the infected xylem bundle a brownish discoloration was found, probably of phenolic origin (Fig. 7D and F). In the stem of susceptible plants, *Fusarium* colonization spreaded from out the xylem vessels into xylem parenchyma cells and cambial cells (Fig. 7E), whereas in resistant plants *Fusarium* was restricted to the xylem vessels and some xylem parenchyma cells (Fig. 7F). These results indicated that there is a co-localization of *I-2* expression (Fig. 6) and the site of defence responses in resistant plants, notably the xylem parenchyma cells and the cambial zone.

### DISCUSSION

Many plant-pathogen interactions fit the gene-for-gene hypothesis (Flor, 1971) which states that plants are resistant when they contain an *R* gene that matches an avirulence *(Avr)* gene in the invading pathogen. Isolation of plant *R* genes and pathogen *Avr* genes has revealed little about the role their gene products play in the defence response. Structural analysis of *R* genes, that share many common features, suggests that they are active in signalling cascade(s) that coordinate initial plant defence responses to impair pathogen growth (Hammond-Kosack and Jones, 1997; Ellis and Jones, 1998). While avirulence genes probably have a role in fitness or pathogenicity of the pathogen (Leach and White, 1996; Vivian and Gibbon, 1997), R gene products may have a function in plant development, and hence are expressed in healthy, unchallenged plants ready to detect the attack (Hammond-Kosack and Jones, 1997).

Detection of *I-2* transcripts using Northern blot analysis appeared to be difficult. However, RT-PCR analysis revealed that *I-2* and several of its homologs are expressed in roots, stems and leaves (Fig. 1B). Because amplifications were not performed quantitatively, band intensity are not likely to reflect actual expression levels, but are rather the result of differences in efficiency at which fragments are amplified. The fact that the level of *I-2* transcripts is very low, that more than one homologs are transcribed, and that based on the known sequences of *I-2* and its homologs no *I-2* specific probes could be designed for *in situ* hybridization, prompted us to use a promoter-*uidA* fusion for *I-2* expression analysis.

The functionality of the *I-2* promoter fragment used was tested by complementation analysis. A construct containing a 3.8 kb 5' flanking sequence of the *I-2* gene upstream of the *I-2* coding and 1.1 kb 3' downstream region conferred resistance to a susceptible line. This indicated that the 3.8 kb promoter of *I-2* is sufficient for functional expression of the *I-2* resistance gene. Only in 29% of the transgenic lines tested complementation was found. Similar functional transformation frequencies were obtained using cosmids containing the *I-2* gene (Simons et al., 1998). In transgenic lines containing the cosmid with the largest promoter sequence (B22), where external influences will be very small, resistance was found in 73% of the transgenic lines. When cosmids were used with shorter promoter sequences, frequencies at which resistant lines were found dropped. For example, with cosmid A55 that contains a promoter sequence of approximately 3 kb, only in 23% of the transgenic lines resistance was found. This suggests that for functional *I-2* expression flanking sequences of the gene are important.

Transgenic plants were generated with the 3.8 kb 5' flanking sequence of the *I-2* gene fused to the GUS reporter gene. Levels of GUS activity in these transgenic plants showed to be highly variable, supporting the observation that the *I-2* promoter activity is sensitive to position effects. The GUS activity found were in agreement with the RT-PCR, namely expression in roots, stems and leaves. RNA gel blot analyses using *RPS2, RPM1, RPP5, Mi, Pto, Prf*, *Xa21* and Cf-9 as probes have revealed the presence of low abundant transcripts in unchallenged plants (Grant et al., 1995; Salemon et al., 1996; Hammond-Kosack and Jones, 1997; Parker et al., 1997; Milligan et al., 1998). This indicates that most *R* genes, and their homologs, are expressed in the absence of the corresponding *avr* expressing pathogen. *I-2* shares this common feature of *R* genes since expression of *I-2* was detected in tomato plants free of *Fusarium.* For most of the *R* genes induction of expression by the pathogen has not been observed or is not known because of the localized response. Only for the nematode resistance genes *Hs*1^{pro-1} and the bacterial resistance gene *Xa*1 induction by the pathogen has been shown (Cai et al., 1997; Yoshimura et al., 1998). In *F*. *oxysporum* f.sp. *lycopersici* infected tomato plants no increased *I-2* promoter driven GUS activity was found (Fig. 4). The same *I-2* promoter-*uidA* constructs were used to transform cell suspension of tomato cell line MSK8. Transgenic cell lines showed constitutively GUS activity; treatment of the cells with conidia of *F*. *oxysporum* f.sp. *lycopersici* race 1 or race 2, culture filtrates of *F. oxysporum* f.sp. *lycopersici* or xylanase (an aspecific elicitor) did not change the GUS activity. Taken together, these results suggest that the *I-2* expression is not regulated by pathogen infection.

Histochemical observations have revealed that GUS activity was primarily found in vascular tissue. Therefore, the relative GUS activity in roots, stems and leaves (Fig. 3) very well could reflect the relative presence of vascular cells in these tissues. The observation that *F. oxysporum* f.sp. *lycopersici* colonizes the vascular tissue of the plant, suggests that *I-2* is expressed in cells that are responsible for the resistance responses. The fungus enters the root via wounds, including natural wounds caused by lateral root formation. At the site of lateral root formation, pronounced *I-2* expression was found in a ring of cells lining at the base of the lateral root, probably outside the vascular tissue of the main root. The fungus that enters the disrupted cortex has to pass this site of *I-2* expression before it invades the vascular tissue. This might create a recognition point for the *I-2* based defences. Growing in the xylem vessels of the root *F. oxysporum* f.sp. *lycopersici* will try to spread lateral and vertical. Vertical invasion is restricted by the production of gels, gums and tyloses. These are produced by xylem parenchyma cells, which *show I-2* expression indicating that the *I-2* gene could directly mediate these responses in xylem parenchyma cells. In the lateral direction it has been proposed that cell wall depositions and the production of antifungal compounds are responsible for the resistance response, and that these resistance reactions are orchestrated by secondary xylem parenchyma cells (Beckman et al., 1989). In susceptible plants *F. oxysporum* f.sp. *lycopersici* invades the xylem parenchyma and even continues into the cambium zone (Fig. 7C and E). Plants of which the cambium zone is affected will no longer be able to bypass the infection by the creation of new xylem vessels. In resistant tomato plants lateral growth of *F. oxysporum* f.sp. *lycopersici* is prevented. This is associated with many defence responses in cells flanking the xylem vessels. Among these responses is a brownish discolourization of phenolic origin (Fig. 7D and F) (Beckman, 1987). GUS activity in xylem parenchyma cells and in cambium cells, as found, overlaps with the location where the crucial resistance mechanism is expected. Expression of *I-2* in the lateral root and adventitious root formation suggests that *I-2* might also be involved in the formation of new roots to compensate the affected absorptive and transport capacity of the root system impaired by *F. oxysporum* f.sp. *lycopersici* infection.

It is speculated that *R* gene products will activate multiple signalling pathways simultaneously such as was found for numerous mammalian receptor proteins (O'Neill, 1995; Li et al., 1997; Ellis and Jones, 1998). Furthermore, sequence homology has been found between NBS containing *R* genes and genes involved in regulation of apoptotic cell death in animals (Chinnaiyan et al., 1997; Van der Biezen and Jones, 1998). This has led to the hypothesis that *R* genes are the key components in a branched signalling pathway resulting in the induction of a wide range of responses that are directed against a broad range of pathogens. One of these branches might include the mediation of rapid host cell death. The resistance of tomato to *F*. *oxysporum* f.sp. *lycopersici* is accompanied by many general defence responses like callose deposition, lignification, production of phytoalexins and induction of PR proteins (Beckman, 1987; Beckman and Roberts, 1995). Since the resistance response is mediated by xylem parenchyma cells, defence mechanisms explored by these cells will probably be close to their normal activity which involves the formation or support of xylem. Tracheary element formation is characterized by secondary wall thickening, which are subsequently lignified, and a novel form of programmed cell death resulting in mature tracheary elements (McCann, 1997; Jones and Groover, 1997). In certain situations like lateral root formation, or vascular obstruction by pathogens, cells that are already differentiated will trans-differentiate to form vascular tissue, thereby establishing continuity of water-transporting tissue (Sachs, 1981; Aloni, 1987). *I-2* expression is found in vascular cambium, xylem parenchyma cells and premature xylem vessels, tissue that can develop into tracheary elements. Furthermore, *I-2* expression was found in lateral and adventitious root formation. This all together let us to hypothesize that the *I-2* gene, or the original function of *I-2* like genes, could be involved in the acceleration of de-differentiation of tissue or in the signal transduction pathway leading to the development of vascular tissue.

### REFERENCES

Aloni, R (1987) Differentiation of vascular tissue. Annu Rev Plant Physiol 38:179-204.

Beckman CM (1987) The nature of wilt disease of plants. APS Press, St. Paul, Minnesota.

Beckman CH, Roberts EM (1995) On the nature and genetic basis for resistance and tolerance to wilt disease of plants. Adv Bot Res 21:35-77.

Beckman CH, Verdier PA, Mueller WC (1989) A system of defence in depth provided by vascular parenchyma cells of tomato in response to vascular infection with *Fusarium oxysporum* f.sp. *lycopersici,* race 1. Physiol and Mol Plant Pathol 34:227-239.

Bishop CD, Cooper RM (1983) An ultrastructural study of root invasion in three vascular wilt diseases. Physiol Plant Pathol 22:15-27.

Cai D, Klein M, Kifle S, Harloff H-J, Sandal NN, Marcker KA, Klein-Lankhorst RM, Salentijn EMJ, Lange W, Stiekema WJ, Wyss U, Grundler FMW, Jung C (1997) Positional cloning of a gene for nematode resistance in sugar beet. Science 275:832-834.

Chinnaiyan AM, Chaudhary D, O'Rourke K, Koonin EV, Dixit VM (1997) Role of CED-4 in the activation of CED-3. Nature 338:728-729.

Ellis J, Jones D (1998) Structure and function of proteins controlling strain-specific pathogen resistance in plants. Curr Opin Plant Biol 1:288-293.

Fillatti JJ, Kiser J, Rose R, Comai L (1987) Efficient transformation of a glyphosate tolerance gene into tomato using a binary Agrobacterium tumefaciens vector. Bio/technology 5:726-730.

Flor HH (1971) Current status of the gene-for-gene concept. Annu Rev Phytopathol 9:275-296.

Grant MR, Godard L, Straube E, Ashfield T, Leward J, Sattler A, Innes RW, Dangl JL (1995) Structure of the Arabidopsis *RPM1* gene enabling dual specificity disease resistance. Science 269:843-846.Jefferson RE, Kavanagh T, Bevan MW (1987) GUS fusions: β-glucuronidase as a sensitive and versatile gene fusion marker in higher plants. EMBO J. 6:3901-3907.

Jones AL, Groover ATG (1997) Programmed cell death of plant tracheary elements differentiating *in vitro.* Protoplasma 196:197:211.

Hammond-Kosack KE, Jones JDG (1997) Plant disease resistance genes. Annu Rev Plant Physiol Plant Mol Biol 48:575-607.

Hutson RA, Smith IM (1983) The response of tomato seedling roots to infection by *Verticillium albo-atrum* or *Fusarium oxysporm* f.sp. *lycopersici.* Annu Appl Biol 102:89-97.

Leach JE, White FF (1996) Bacterial avirulence genes. Annu Rev Phytopathol 34:153-179.

McCann MC (1997) Tracheary element formation: building up to a dead end. Trends in plant Science 2:333-338.

Mes JJ, Weststeijn EA, Herlaar F, Lambalk JJM, Wijbrandi J, Haring MA, Comelissen BJC (1999) Biological and molecular characterization of *Fusarium oxysporum* f.sp. *lycopersici* divides race 1 into separate virulence groups. Phytopathology, in press.

Milligan SB, Bodeau J, Yaghoobi J, Kaloshian I, Zabel P, Williamson VM (1998) The root knot resistance gene Mi from tomato is a member of the leucine zipper, nucleotide binding, leucine-rich repeat family of plant genes. Plant Cell 10:1307-1319.

O'Neill LA (1995) Interleukin-1 signal transduction. Int Clin Lab Res 25:169-177.

Ori N, Eshed Y, Paran I, Presting G, Aviv D, Tanksley S, Zamir D, Fluhr R (1997) The *I2C* family from the wilt disease resistance locus I2 belongs to the nucleotide binding, leucine-rich repeat superfamily of plant resistance genes. Plant Cell 9:521-532.

Parker JE, Coleman MJ, Szabo V, Frost LN, Schmidt R, Van der Biezen EA, Moores T, Dean C, Daniels MJ, Jones JDG (1997) The Arabidopsis downy mildew resistance gene *RPP5* shares similarity to the toll interleukine-1 receptors with *N* and L6. Plant Cell 9:879-894.

Roberts IN, Oliver RP, Punt PJ, Van den Hondel CAMJJ (1989) Expression of the *Escherichia coli* β-glucuronidase gene in industrial and phytopathogenic filamentous fungi. Curr Genet 15:177-180.

Sachs T (1981) The controle of patterned differentation of vascular tissue. Adv Bot Res 9:151-262.

Salmeron JM, Oldroyd GED, Rommens CMT, Scofield SR, Kim H-S, Lavelle DT, Dahlbeck D, Staskawicz BJ (1996) Tomato *Prf* is a member of a leucine-rich repeat class of plant disease resistance genes and lies embedded within the Pto kinase gene cluster. Cell 86:123-133.

Simons G, Groenendijk J, Wijbrandi J, Reijans M, Groenen J, Diergaarde P, Van der Lee T, Bleeker M, Onstenk J, De Both M, Haring M, Mes J, Cornelissen B, Zabeau M, Vos P (1998) Dissection of the Fusarium *I2* gene cluster in tomato reveals six homologs and one active gene copy. Plant Cell 10:1055-1068.

Song W-Y, Wang G-L, Chen L-L, Kim H-S, Pi L-Y, Holsten T, Gardner J, Wang B, Zhai W-X, Zhu L-H, Fauquet C, Ronald P (1995) A receptor kinase-like protein encoded by the rice disease resistence gene, *Xa21.* Science 270:1804-1806.

Toriyama K, Thorsness MK, Nasrallah JB, Nasrallah ME (1991) A Brassica S locus gene promoter directs sporophytic expression in the anther tapetum of transgenic Arabidopsis. Dev Biol 143:427-31.

Van der Biezen EA, Jones JDG (1998) The NB-ARC domain: a novel signalling motif shared by plant resistance gene products and regulators of cell death in animals. Curr Biol 8:226-227.

Vivian A, Gibbon MJ (1997) Avirulence genes in plant-pathogenic bacteria: signals or weapons, Microbiology 143:693-701.

Yoshimura S, Yamanouchi U, Katayose Y, Toki S, Wang ZX, Kono I, Kurata N, Yano M, Iwata N, Sasaki T (1998) Expression *of Xa1,* a bacterial blight-resistance gene in rice, is induced by bacterial inoculation. Proc Natl Acad Sci USA 95:1663-1668.

## Claims

1. Nucleotide sequence having a regulatory activity on the transcription of the 1-2 resistance gene in determined plant host cells, which nucleotide sequence comprises SEQUENCE ID NO:1.

2. Nucleotide sequence according to claim 1 which is SEQUENCE ID NO:1.

3. Regulatory nucleotide sequence according to claim 1 or 2 or a fragment thereof capable of driving a tissue specific transcription of a determined nucleic acid sequence placed under its control.

4. Recombinant nucleotide sequence comprising a nucleotide sequence according to claim 1 or 2 functionally linked to a determined nucleic acid sequence.

5. Recombinant nucleotide sequence according to claim 4, wherein the nucleic acid sequence functionally linked is a sequence encoding the I-2 resistance polypeptide.

6. Recombinant nucleotide sequence according to claim 4 or 5, which is transcribed in a tissue specific manner in plant host cells.

7. Recombinant nucleotide sequence according to anyone of claims 4 to 6, wherein the nucleic acid sequence functionally linked to the regulatory sequence is chosen among sequences expressed in a tissue specific manner in their natural hosts.

8. Recombinant nucleotide sequence according to anyone of claims 4 to 7, which is expressed in a tissue specific manner when it is transferred into a plant.

9. Recombinant nucleotide sequence according to anyone of claims 4 to 8, which is capable of being expressed in plant vascular tissue.

10. Recombinant nucleotide sequence according to anyone of claims 4 to 8, which is capable of being expressed in plant lateral root meristem.

11. Recombinant nucleotide sequence according to anyone of claims 4 to 10 which further comprises a 3' non translated sequence comprising a transcription termination codon.

12. Recombinant nucleotide sequence according to anyone of claims 4 to 11, wherein the nucleic acid sequence functionally linked is a sequence of a gene having activity in mediation of resistance to a plant pathogen when the recombinant nucleotide sequence is operatively transferred to a host plant susceptible to said plant pathogen.

13. Recombinant nucleotide sequence according to claim 12, wherein the plant pathogen is a root pathogen.

14. Recombinant nucleotide sequence according to anyone of claims 12 to 13 wherein the plant pathogen is a fungus, especially of *Fusarium* species.

15. Recombinant vector comprising a nucleotide sequence according to anyone of claims 1 to 3 or recombinant nucleotide sequence according to anyone of claims 4 to 14.

16. Recombinant vector according to claim 15, which is suitable for transforming plant cells or plants.

17. Recombinant cell comprising a nucleotide sequence according to anyone of claims 1 to 3 or recombinant nucleotide sequence according to anyone of claims 4 to 14 or a recombinant vector according to claim 15 or 16.

18. Recombinant cell according to claim 17, which is a plant cell.

19. Recombinant cell according to claim 18, which is a plant root cell or a stem cell or a leaf cell.

20. Plant which is recombined with a nucleotide sequence according to anyone of claims 1 to 3 or a recombinant nucleotide sequence according to anyone of claims 4 to 14 or a vector according to claims 15 or 16.

21. Recombinant plant according to claim 20 which is capable of expressing a gene mediating resistance to a plant pathogen chosen among fungi, in a tissue specific manner.

22. Recombinant plant according to claim 20, which is capable of preventing the infection by a plant pathogen chosen among fungi.

23. Recombinant plant according to anyone of claims 20 to 22, which is a tomato, egg plant, potato, melon, tobacco, Arabidopsis.

24. Use of a nucleotide sequence according to anyone of claims 1 to 14 or of a vector according to anyone of claims 15 or 16 or use of a part of said nucleotide sequence for the tissue specific regulation of the transcription of the determined nucleic acid sequence placed under its control.

25. Use according to claim 24 wherein the nucleotide sequence having a transcription regulatory activity is SEQUENCE ID NO: 2 or a fragment thereof sufficient to drive the transcription of a determined nucleic acid sequence placed under its control.

26. Use according to claim 24 wherein the nucleotide sequence having a transcription regulatory activity is all or part of SEQUENCE ID NO: 3.

27. Use according to anyone of claims 24 to 26, wherein the transcription regulatory activity is obtained in vascular tissue.

28. Use according to anyone of claims 24 to 26, wherein the transcription regulatory activity is obtained in the lateral root meristem tissue.

29. Process for obtaining plants having reduced susceptibility to a plant pathogen, comprising the following steps:
i) inserting into the genome of a plant cell a nucleotide sequence according to anyone of claims 1 to 14, or a vector according to any of claims 15 or 16,
ii) obtaining transformed plant cells,
iii) regenerating from said transformed plant cells genetically transformed plants, and
iv) optionally, propagating said plants.

30. Process according to claim 24 further comprising selecting transformed plants having reduced susceptibility to said plant pathogen.

31. Process for protecting plants in cultivation against plant pathogen infection, which comprises:
i) providing the genome of plants with a nucleotide sequence according to anyone of claims 1 to 14, or a vector according to any of claims 15 or 16, and
ii) growing said plants.
